(19) Europäisches Patentamt
European Patent Office
Office européen des brevets

(11) EP 3 657 167 A1

(12) EUROPEAN PATENT APPLICATION

(43) Date of publication:
**27.05.2020 Bulletin 2020/22**

(51) Int Cl.:
***G01N 33/50*** *(2006.01)* ***G01N 33/574*** *(2006.01)*

(21) Application number: **18207636.4**

(22) Date of filing: **21.11.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Stichting Het Nederlands Kanker Instituut-**
**Antoni van Leeuwenhoek Ziekenhuis**
**1066 CX Amsterdam (NL)**

(72) Inventors:
- **OOFT, Salo Noël**
  **1066 CX AMSTERDAM (NL)**
- **VOEST, Emile Eugene**
  **1066 CX AMSTERDAM (NL)**

(74) Representative: **Algemeen Octrooi- en Merkenbureau B.V.**
**P.O. Box 645**
**5600 AP Eindhoven (NL)**

# (54) METHOD OF PREDICTING RESPONSIVENESS OF A GASTROINTESTINAL CANCER PATIENT TO A CHEMOTHERAPY TREATMENT

(57) Provided are methods for predicting the responsiveness (predictive assays) of a gastrointestinal cancer patient or stratifying said patient into a responder or non-responder to a chemotherapy treatment comprising: 1) 5-flurouracil or capecitabine in combination with irinotecan or 2) irinotecan only. Also provided are methods of treating a gastrointestinal cancer patient using the predictive assay of the invention. The methods of the invention can be advantageously used to decide on or assign the best treatment option(s) to a gastrointestinal cancer patient, to prevent exposing said patient to the unnecessary side effects associated with a treatment which will prove to be ineffective at a later stage, and/or to reduce the financial burden associated with ineffective cancer treatment options.

Fig. 1A

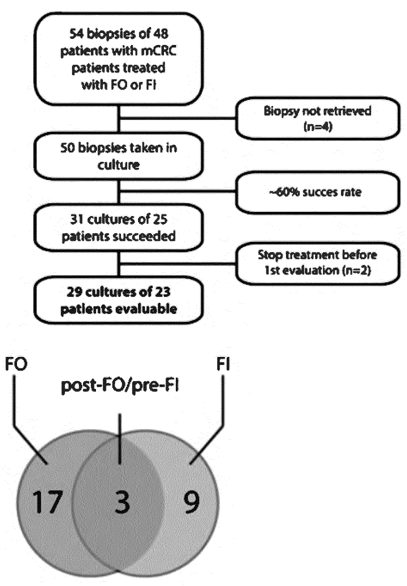


EP 3 657 167 A1

Fig. 1B

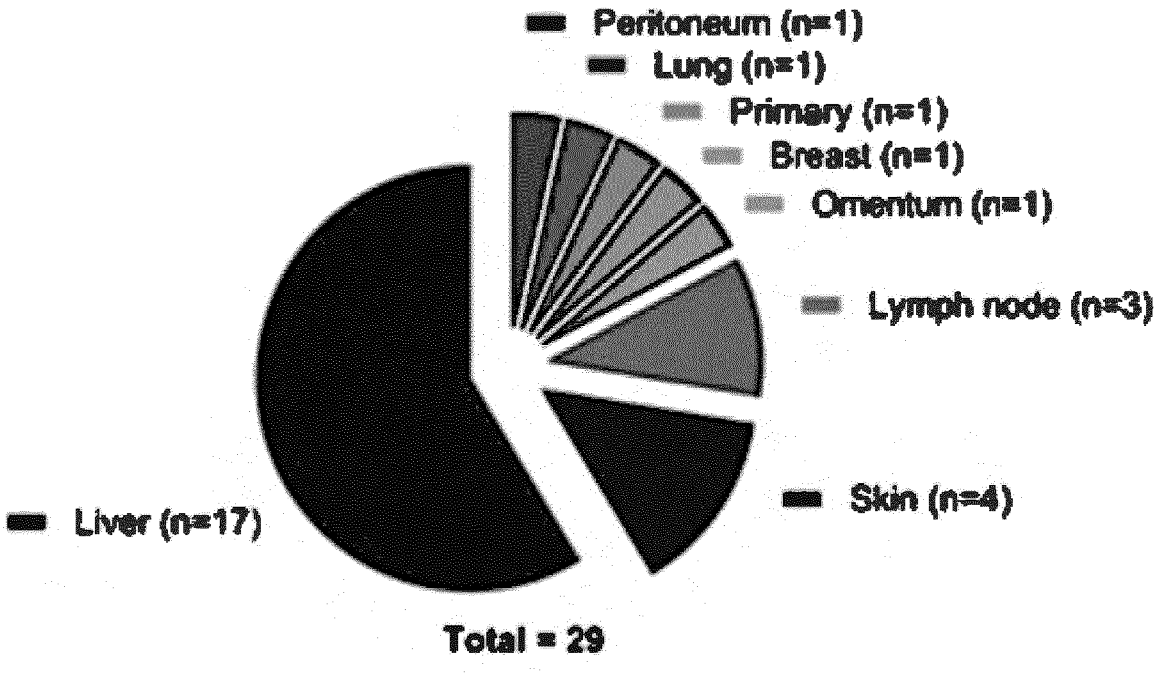
Peritoneum (n=1)
Lung (n=1)
Primary (n=1)
Breast (n=1)
Omentum (n=1)
Lymph node (n=3)
Skin (n=4)
Liver (n=17)
Total = 29

Fig. 1C

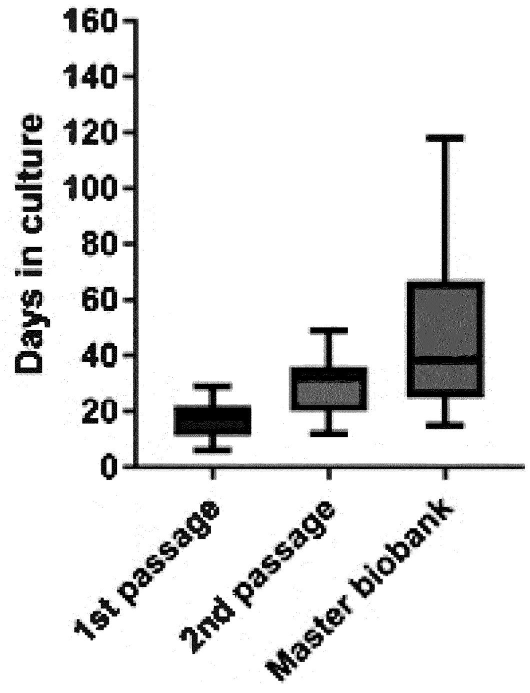
Days in culture
0
20
40
60
80
100
120
140
160
1st passage
2nd passage
Master biobank

## Description

### FIELD OF THE INVENTION

The present invention relates to the field of cancer and chemotherapy treatment, more particularly to the field of gastrointestinal cancer such as colorectal cancer. Provided are methods for predicting the responsiveness (predictive assays) of a gastrointestinal cancer patient or stratifying said patient into a responder or non-responder to a chemotherapy treatment comprising: 1) 5-flurouracil or capecitabine in combination with irinotecan or 2) irinotecan only. Also provided are methods of treating a gastrointestinal cancer patient using the predictive assay of the invention. The methods of the invention can be advantageously used to decide on or assign the best treatment option(s) to a gastrointestinal cancer patient, to prevent exposing said patient to the unnecessary side effects associated with a treatment which will prove to be ineffective at a later stage, and/or to reduce the financial burden associated with ineffective cancer treatment options.

### BACKGROUND OF THE INVENTION

Cancer is a leading cause of death worldwide, accounting for more than 8.8 million deaths in 2015 (statistics from World Health organization (WTO)). Cancer can be generally defined as a group of diseases involving abnormal cell growth. Generally, when cancer develops, normal cells become progressively abnormal over time as they acquire mutations which allow them to escape immune surveillance, survive, grow uncontrollably, and spread through the body (Lazebnik, Y., (2010), Nature Reviews Cancer, Vol. 10, pages 232-233; Bekele and Brindley (2012), Clinical Lipidology, Vol.7, pages 313-328).

Chemotherapy (also known as "chemo") represents one of the most common treatment for cancer. Chemotherapy is the systemic treatment of cancer with anticancer drugs, which aim to kill cancer cells or poison cancer cells to inhibit cell division or mitosis.

Chemotherapy uses one or more anti-cancer drugs (also known as chemotherapeutic agents) as part of a standardized chemotherapy regimen. The efficacy of chemotherapy depends on the type of cancer and the stage. The overall effectiveness ranges from being curative (e.g. certain cancers such as leukemia) to being ineffective. Further, chemotherapy treatments are associated with severe side effects. The most common chemotherapy medications affect mainly the fast-dividing cells (normal and cancerous cells) of the body, such as blood cells and the cells lining the mouth, stomach, and intestines. Nausea, vomiting, anorexia, diarrhoea, abdominal cramps, constipation, and hair loss are common side-effects of chemotherapeutic medications that kill fast-dividing cells. Chemotherapy-related toxicities can occur acutely after administration, within hours or days, or chronically, from weeks to years.

Another notorious side effects of chemotherapy, among many others, include destruction of bone marrow cells, which ultimately leads to decreased white blood cells, red blood cells, and platelets counts in the blood. This can lead to the development of anemia, fatigue, and compromised immune system response, among others conditions.

Although chemotherapy has proven to be useful (e.g. improves the life of cancer patients, at least for some time), it does not always work in completely destroying the cancer. Further, not all cancer patients respond well to a given chemotherapy regimen prescribed for a particular cancer type (e.g. colorectal cancer) because in some patients, the chosen chemotherapeutic treatment is not efficient in eliminating or reducing the size of tumor and/or side effects are too debilitating or intolerable. Resistance to a given chemotherapy treatment, which ultimately leads to treatment failure in certain patients, represents another main limitation associated with chemotherapy.

In the absence of predictive markers for predicting responsiveness to a given chemotherapy treatment, patients with cancer are frequently treated or over-treated with chemotherapeutic agent(s) which have little or no therapeutic benefits while at the same time suffering from serious side effects such as those described above. In other words, the patient finds him/herself in a trial-and-error situation to establish an effective treatment option, which may take too long or may be too harmful.

One particular example of a type of cancer which is often treated with one or more chemotherapeutic agents is gastrointestinal cancer such as for instance, colorectal cancer, esophagus cancer, stomach cancer, biliary system cancer, small intestine cancer, large intestine cancer (or colon cancer), rectum cancer and anus cancer.

In the case of colorectal cancer, particularly in metastatic colorectal cancer, the standard of care therapy involves administration of one or more of the following chemotherapeutic agents including, for instance, capecitabine (Xeloda), fluorouracil (5-FU (Adrucil), irinotecan (Camptosar), oxaliplatin (Eloxatin), and trifluridine/tipiracil (TAS-102, Lonsurf).

In some cases, treatment regimens (e.g. combination of two or more drugs) are prescribed. Examples of such treatment regimens include for instance: 1) 5-FU in combination with leucovorin, which is a vitamin that improves the effectiveness of 5-FU; 2) capecitabine in combination with 5-FU; 3) 5-FU in combination with leucovorin and oxaliplatin (abbreviated as FOLFOX); 4) 5-FU in combination with leucovorin and irinotecan (abbreviated FOLFIRI); 5) 5-FU or capecitabine in combination with irinotecan, and 6) 5-FU or capecitabine in combination with oxaliplatin. In certain cases, irinotecan is administered alone.

However, the standard of care chemotherapy treatment options described above do not work or are not effective in all patients, e.g. some colorectal cancer patients do not respond to such chemotherapy treatment, and as a result said patients suffer from unnecessary side effects (as described above), are deprived of an effective treatment, and are losing precious time in their battle against cancer.

Because the standard of care chemotherapy for gastrointestinal cancer (e.g. colorectal cancer) is typically administered as combinations of two or more drugs, each with a broad mechanism of action, the development of predictive algorithms and in vitro modeling is highly complex, and has not (yet) yielded predictive tests for this type of cancer.

Other predictive markers (gene markers) or methods relying on the use of genomic information have been shown to facilitate patient selection for targeted therapies with drugs acting on specific molecular targets that are associated with cancer such as ***EGFR inhibitors*** (e.g. gefitinib, erlotinib, afatinib, brigatinib and icotinib, cetuximab, dacomatinib, and others), ***MEK inhibitors*** (e.g. trametinib, binimetinib, selumetinib, PD-325901, and others), ***BRAF inhibitors*** (e.g. vemurafenib, dabrafenib, LGX818, and others) or ***monoclonal antibodies*** (e.g. rituximab, trastuzumab, alemtuzumab, cetuximab, panitumumab, ipilimumab, and many others).

However, the genomic-based strategies have been unsuccessful for chemotherapy, mainly because of a lack of understanding of the diverse mechanism of action involved. Hence, although many studies have attempted to identify predictive markers, this has not resulted in a clinically meaningful way to select patients for chemotherapy treatment. For instance a method relying on the use of the Werner syndrome ATP-dependent helicase (WRN) promoter hypermethylation status failed to predict favorable outcomes for metastatic colorectal cancer patients treated with irinotecan-based therapy (see Bosch et al (2016), Clin. Cancer Res., Vol: 22, pages 4612-22).

Previous attempts to use patient material *ex vivo* to determine responsiveness to chemotherapy have had limited success due to the low success rate of establishing cell lines or the time to generate sufficient material when generating patient derived xenografts (PDXs). For instance, it takes on average 3-4 months to generate PDX material from a patient before it can be used from predicting individual response to a given treatment. Therefore such long duration has substantially limited use of PDX-derived models for personalized response prediction.

Taken together, personalized cancer treatment, particularly gastrointestinal cancer treatment (e.g. colorectal cancer), in the context of chemotherapy (using one or two chemotherapeutic agents or more) is currently infeasible and novel predictive assays, to identify effective drugs for individual patients, are needed.

In light of this, methods for better identifying or selecting or stratifying gastrointestinal cancer patients, particularly colorectal cancer patients, into responders or non-responders to a standard of care chemotherapeutic treatment such as those described above would be highly desirable but are not yet readily available.

In particular there is a clear need in the art for reliable, efficient and reproducible (standardized) methods for better identifying or selecting or stratifying gastrointestinal cancer patients, particularly colorectal cancer patients, into responders or non-responders to a standard of care chemotherapeutic treatment such as those described above. Accordingly, the technical problem underlying the present invention can been seen in the provision of such methods. The technical problem is solved by the embodiments characterized in the claims and herein below.

## SUMMARY OF THE INVENTION

The present inventors have surprisingly found a reliable way to predict responsiveness (or treatment outcome) of a gastrointestinal cancer patient (e.g. colorectal cancer patient or pancreatic cancer patient or others) to a chemotherapeutic treatment comprising: 1) 5-FU or capecitabine in combination with irinotecan, or 2) irinotecan alone.

The method of the invention relies on the use of patient-derived tumor organoid (PDO), which can be generated in a relatively shorter period of time (e.g. within 2-4 weeks) compared to other model such as patient derived xenografts (PDXs) models (3-4 months generation time). The present inventors have found that by measuring the growth rate (as taught herein) of the PDOs in response to 1) 5-FU or capecitabine in combination with irinotecan, or 2) irinotecan alone, one can determine (based on the use of a reference value) the responsiveness of a patient (from which the PDO are derived) to said chemotherapeutic treatment in vitro, in a non-invasive manner, prior actually treating (in vivo) said patient with the chemotherapeutic treatment.

In other words, the present inventors found that based on the drug-induced growth rate inhibition (GR) (score) (and threshold value or reference value (REF) derived thereof, as explained herein) of the PDOs (in vitro) in response to being exposed to either 1) 5-FU or capecitabine (oral pro-drug of 5-FU) in combination with irinotecan, or 2) irinotecan alone for a given length of time (at least 3 hours), one can accurately or reliably predict whether the patient from whom the PDO is derived is a responder or a non-responder to the chemotherapeutic treatment comprising: 1) 5-FU or capecitabine in combination with irinotecan, or 2) irinotecan alone.

In other words, the present invention provides a new, reliable *in vitro* method for predicting the clinical response of a gastrointestinal cancer patient to the chemotherapeutic treatment with irinotecan alone or irinotecan in combination with 5-FU or capecitabine. Furthermore, this method can provide such information faster (e.g. as quickly as for example to 2-4 weeks in comparison to 3-4 months with PDX) than other existing methods such as patient-derived xenograft-based techniques (e.g. PDX models, as explained above). This represents a tangible advantage for the patients, e.g. provides a test which can be readily carried out by clinicians in hospital setting in a relatively short period of time, which saves precious time in deciding the proper treatment course for a patient.

Prior the present invention, methods for predicting clinical response of a gastrointestinal cancer patient to chemotherapeutic treatment in general but more particularly to a chemotherapeutic treatment comprising irinotecan alone or irinotecan in combination with 5-FU or capecitabine were not available. As a consequence, gastrointestinal (e.g. colorectal cancer or pancreatic cancer patient or others) cancer patients who turned out to be non-responder to such therapy were frequently treated or received inadequate or ineffective standard of care chemotherapy, which in turn caused severe side effects and no or little improvements in health or quality of life. The present inventors found a solution to this problem by providing the methods of the invention as described below. /

## DETAILED DESCRIPTION OF THE INVENTION

### Definitions

The section headings used herein are for organizational purposes only and are not to be construed as limiting the subject matter described.

A portion of this disclosure contains material that is subject to copyright protection (such as, but not limited to, diagrams, device photographs, or any other aspects of this submission for which copyright protection is or may be available in any jurisdiction.). The copyright owner has no objection to the facsimile reproduction by anyone of the patent document or patent disclosure, as it appears in the Patent Office patent file or records, but otherwise reserves all copyright rights whatsoever.

Various terms relating to the methods, compositions, uses and other aspects of the present invention are used throughout the specification and claims. Such terms are to be given their ordinary meaning in the art to which the invention pertains, unless otherwise indicated. Other specifically defined terms are to be construed in a manner consistent with the definition provided herein. Although any methods and materials similar or equivalent to those described herein can be used in the practice for testing of the present invention, the preferred materials and methods are described herein.

For purposes of the present invention, the following terms are defined below.

As used herein, the singular forms "a," "an" and "the" include plural referents unless the context clearly dictates otherwise. For example, a method for administrating a drug includes the administrating of a plurality of molecules (e.g. 10’s, 100’s, 1000’s, 10’s of thousands, 100’s of thousands, millions, or more molecules).

As used herein, the term "and/or" indicates that one or more of the stated cases may occur, alone or in combination with at least one of the stated cases, up to with all of the stated cases.

As used herein, the term "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc.

The term "to comprise" and its conjugations as used herein is used in its non-limiting sense to mean that items following the word are included, but items not specifically mentioned are not excluded. It also encompasses the more limiting "to consist of"."

The term "subject" or "patient" (used interchangeably) as used herein refers to a human subject male or female, adult, child or infant, suffering from a gastrointestinal cancer (e.g. colorecal), regardless of the stage or state of the cancer.

The terms "cancer" and "tumor" (used interchangeably), as used herein, refer to or describe the physiological condition in humans that is typically characterized by unregulated cell growth. The terms "cancer" and "tumor" also refer to cells that have undergone a malignant transformation that makes them pathological to the host organism. Cancer cells can be distinguished from non-cancerous cells by techniques known to the skilled person.

The term "gastrointestinal cancer" as used herein refers to malignant conditions of the gastrointestinal tract (GI tract) and accessory organs of digestion, including the esophagus cancer, stomach cancer, biliary system cancer, small intestine cancer, large intestine (colon) cancer, colorectal cancer, pancreatic cancer, rectum cancer and anus cancer. The symptoms relate to the organ affected and can include obstruction (leading to difficulty swallowing or defecating), abnormal bleeding or other associated problems. The diagnosis often requires endoscopy, followed by biopsy of suspicious tissue. The treatment depends on the location of the tumor, as well as the type of cancer cell and whether it has invaded other tissues or spread elsewhere (metastasis).

The term "colorectal cancer" (abbreviated CRC) as used herein refers to a cancer arising from uncontrolled cell growth in the colon, rectum or in the appendix. Colon and rectal tumors have similar aetiology (e.g. initiation, development) the same type cancer. There are several subtypes known in CRC, with distinct genetic origins (e.g. see Yang Liu et al (2018), Cancer cell, Vol. 33, pages 721-35). Symptoms of colorectal cancer typically include rectal bleeding, anemia which are sometimes associated with weight loss and changes in bowel habits. Colorectal cancer typically starts in the lining of the bowel and if left untreated, can grow into the muscle layers underneath, and then through the bowel wall. Cancers that are confined within the wall of the colon are often curable with surgery while cancer that has spread

widely around the body (metastasis) is usually not curable and management then focuses on extending the person's life via chemotherapy and improving quality of life.

Different types of treatment are available for patients with gastrointestinal cancer (e.g. colorectal cancer or pancreatic cancer or others). Specifically, there are four main types of standard of care treatments for gastrointestinal cancer including: 1) surgery, 2) chemotherapy, 3) radiation therapy and 4) targeted therapy with the EGFR inhibitor such as cetuximab.

For instance, chemotherapy is frequently used to treat gastrointestinal cancer, particularly colorectal cancer. The standard of care chemotherapy treatment includes administration of one or more of the following chemotherapeutic agents including, for instance, capecitabine (Xeloda), fluorouracil (5-FU (Adrucil), irinotecan (Camptosar), oxaliplatin (Eloxatin), and trifluridine/tipiracil (TAS-102, Lonsurf). In some cases, the following combination treatments are prescribed including for instance: 1) 5-FU in combination with folinic acid (leucovorin), which is a vitamin that improves the effectiveness of 5-FU, 2) capecitabine in combination with 5-FU, 3) 5-FU in combination with folinic acid (leucovorin) and oxaliplatin (abbreviated as FOLFOX), 4) 5-FU in combination with folinic acid (leucovorin) and irinotecan (abbreviated FOLFIRI), 5) 5-FU or capecitabine in combination with irinotecan, and 6) 5-FU or capecitabine in combination with oxaliplatin. In other situations, treatment with Irinotecan alone is prescribed.

While all treatment options above can produce responses in patients with advanced disease (e.g. colorectal cancer or pancreatic cancer or others), none were found to be curative beyond surgery in early stage of disease. Notably, some patients demonstrate pre-existing resistance to certain of these therapies in particular to chemotherapeutic treatment comprising 5-FU in combination with irinotecan with or without (leucovorin) (often abbreviated as FOLFIRI therapy). Thus only a fraction of gastrointestinal cancer patients, particularly colorectal cancer patients, respond well to chemotherapy. As such, gastrointestinal cancer, particularly colorectal cancer continues to be a major cause of cancer mortality, and personalized treatment decisions are still needed, i.e. methods which allow one to assign a chemotherapeutic treatment to a cancer patient which have been identified as a responder to said chemotherapeutic treatment and methods which allow one to avoid assigning a chemotherapeutic treatment to a gastrointestinal cancer patient which have been identified as a non-responder to said chemotherapeutic treatment. The methods of the present inventions as taught herein, fulfil this need.

The terms "treat," treating", "treatment", "therapy" and the like as used herein refer to reducing or ameliorating a disorder (e.g. cancer) and/or symptoms associated therewith. It is appreciated that treating a disorder or condition (e.g. gastrointestinal cancer such as colorectal cancer) does not require that the disorder, condition or symptoms associated therewith be completely eliminated. It is further understood that the terms "treat," treating", "treatment", "therapy" and as used herein may be a first or first line of treatment (i.e. patient is naive to any cancer treatment) or a second or third line treatment and so on (i.e. the first treatment or second treatment and so on was not effective or has failed).

The term "patient-derived tumor organoid (abbreviated PDO)" as used herein refers to an in vitro 3D cell-based scaffolding technology that enables patient-derived cancer cells such as epithelial tumor cells (primary cells) to be cultured in laboratories in a 3D matrix to recapitulate the biology (genetic and physical and morphological features) of the parent tissue, such as cancer tissue. PDOs can be subsequently used for testing against a panel of approved drugs and new drug candidates or for testing *ex vivo* drug sensitivity. PDOs are often referred to as "mini organs" because they share many structural features of the organ or tissue of interest (e.g. cancer) including cell type, cell-cell junctions, cell polarity, adhesion-dependent cell communication and interactions with a complex extracellular matrix (ECM), and others. PDOs can be obtained using standard methods in the art such as for instance the method described in Wetering et al (2015), Cell, Vol: 161 (4), pages 933-945; Weeber et al (2015) PNAS, Vol:112 (43), pages 13308-13311; Vlachogiannis, G., et al (2018), Science, Vol: 359, pages 920-926; and Tiriac, H., et al (2018), Cancer Discov., Vol:8(9); pages 1-18, doi:10.1158/2159-8290.CD-18-0349) or as described herein in the example section.

The term "chemotherapeutic treatment" or "chemotherapy" (often abbreviated as "chemo") as used herein refers to a drug or a combination of drugs in a cancer treatment, e.g. which can be part part of a standardized chemotherapy regimen, for instance as described above. Chemotherapy may be given with a curative intent (which almost always involves combinations of drugs), or it may aim to prolong life or to reduce symptoms (palliative chemotherapy). Chemotherapy is often used, either as the primary form of treatment or as a supplement to other treatments, to treat patients with cancer that has spread (metastasized) from the place in the body where it started. Chemotherapy may also be used the keep cancer from coming back (adjuvant therapy). In the context of the present invention, the term "chemotherapeutic treatment comprising 5-fluorouracil or capecitabine in combination with irinotecan" or a "chemotherapeutic treatment comprising irinotecan alone" refers to the use of: 1) 5-fluorouracil or capecitabine in combination with irinotecan or 2) irinotecan alone.

The term "chemotherapeutic agent(s) or drug(s) (also known as cytotoxic agents) as used herein refers to drugs capable of acting on or targeting rapidly dividing normal (non-cancer cells) and cancerous cells. Several types of chemotherapeutic agents exist including ***alkylating agents*** (e.g. altretamine, busulfan, carboplatin, carmustine, chlorambucil, cisplatin, cyclophosphamide, dacarbazine, lomustine, melphalan, oxaliplatin, temozolomide, thiotepa, and others), ***antimetabolite agents*** (e.g. 5-fluorouracil (5-FU), 6-mercaptopurine (6-MP), capecitabine (Xeloda), cytarabine

(Ara-C), floxuridine, fludarabine, gemcitabine (Gemzar), hydroxycarbamide and others), ***anti-microtubule agents*** (e.g. paclitaxel (Taxol), vinorelbine (Navelbine), docetaxel (Taxotere), vinblastine (Velban), and many others), ***topoisomerase inhibitors type I and II*** (type I: e.g. irinotecan, topotecan, camptothecin and lamellarin D, and others; and type II: e.g. etoposide (VP-16), teniposide, doxorubicin, daunorubicin, mitoxantrone, amsacrine, ellipticines, aurintricarboxylic acid, HU-331, and others), and ***cytotoxic antibiotic agents*** (e.g. dactinomycin, bleomycin, daunorubicin, and others), and many others.

The term "5-fluorouracil" or "fluorouracil" (abbreviated 5-FU or F)" as used herein refers to a chemotherapeutic drug or agent that is well-known by the skilled person. Any source of 5-FU may be used in the present invention. For instance a non-limiting example of 5-FU may be 5-FU sold under the brand name Adrucil. 5-FU is commonly prescribed for the treatment of gastrointestinal cancer such as colorectal cancer. 5-FU acts in several ways, but principally as a thymidylate synthase (TS) inhibitor. Interrupting the action of this enzyme blocks synthesis of the pyrimidine thymidine, which is a nucleoside required for DNA replication. Thymidylate synthase methylates deoxyuridine monophosphate (dUMP) to form thymidine monophosphate (dTMP). Administration of 5-FU causes a scarcity in dTMP, so rapidly dividing cancerous cells undergo cell death via thymineless death. Calcium folinate (also known as leucovorin) provides an exogenous source of reduced folinates and hence stabilises the 5-FU-TS complex, hence enhancing 5-FU's cytotoxicity. 5-FU has the chemical formula $C_4H_3FN_2O_2$ and the following chemical structure (schematic A) (ChEMBL:CHEMBL185; DRUGBANK accession number DB00544 / APRD00516, EXPT03204):

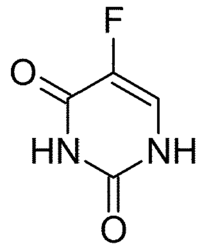

**Schematic A**. 5-fluorouracil (5-FU) chemical structure

The term "capecitabine" as used herein refers to a chemotherapeutic agent or drug that is well-known by the skilled person. Any source of capecitabine may be used in the present invention. For instance, a non-limiting example of capecitabine may be capecitabine which is sold under the brand name Xeloda. Capecitabine is the oral pro-drug of 5-FU. Capecitabine is commonly prescribed for the treatment of gastrointestinal cancer such as colorectal cancer. Once in the body, capecitabine is metabolised to 5-FU, which in turn is a thymidylate synthase inhibitor, hence inhibiting the synthesis of thymidine monophosphate (ThMP), the active form of thymidine which is required for the de novo synthesis of DNA (as explained above for 5-FU). Capecitabine has the chemical formula $C_{15}H_{22}FN_3O_6$ and the following chemical structure (schematic B) (ChEMBL: CHEMBL1773; DRUGBANK accession number DB01101 / APRD00203):

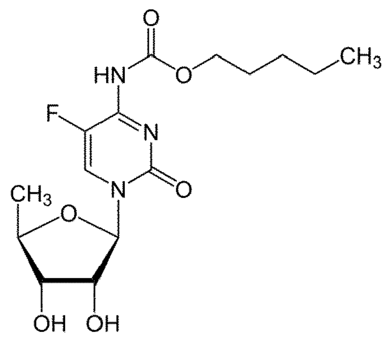

**Schematic B**. Capecitabine chemical structure

The term "irinotecan" (abbreviated as IRI) as used herein refers to a chemotherapeutic agent or drug that is well-known by the skilled person. Any source of irinotecan may be used in the present invention. For instance, a non-limiting example of irinotecan may be irinotecan which is for instance sold under the brand name Camptosar. Irinotecan

is commonly prescribed for the treatment of gastrointestinal cancer such as colorectal cancer.

Irinotecan acts as follows: it is activated by hydrolysis to SN-38, an inhibitor of topoisomerase I. This is then inactivated by glucuronidation by uridine diphosphate glucuronosyltransferase 1A1 (UGT1A1). The inhibition of topoisomerase I by the active metabolite SN-38 eventually leads to inhibition of both DNA replication and transcription. The molecular action of irinotecan occurs by trapping a subset of topoisomerase-1-DNA cleavage complexes, those with a guanine +1 in the DNA sequence. The irinotecan molecule stacks against the base pairs flanking the topoisomerase-induced cleavage site and poisons (inactivates) the topoisomerase 1 enzyme. Irinotecan has the chemical formula $C_{33}H_{38}N_4O_6$ and the following chemical structure (schematic C) (ChEMBL:CHEMBL481: DRUGBANK accession number DB00762 / APRD00579):

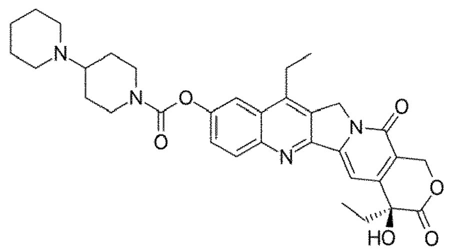

**Schematic C**. Irinotecan chemical structure

The term "bevacizumab" as used herein refers to a therapeutic agent used in the treatment of many cancers (e.g. colorectal cancers, brain cancer, lung cancer, renal cancer, breast cancer, ovarian cancer and others). Bevacizumab is well-known by the skilled person. Any source of bevacizumab may be used in the present invention. For instance, a non-limiting example of bevacizumab may be bevacizumab which sold under the brand name Avastin. Bevacizumab is a recombinant humanized monoclonal antibody that blocks angiogenesis by inhibiting vascular endothelial growth factor A (VEGF-A)). Bevacizumab was approved by the FDA in February 2004 for use in metastatic colorectal cancer when used with standard chemotherapy treatment (as first-line treatment) and with 5-fluorouracil-based therapy for second-line metastatic colorectal cancer. Bevacizumab has the chemical formula $C_{6638}H_{10160}N_{1720}O_{2108}S_{44}$ (CHEMBL1201583; DRUGBANK Accession Number DB00112 (BTD00087, BIOD00087)):

The term "folinic acid" (also known as leucovorin) as used herein refers to a medication used to decrease the toxic effects of other medications such as for instance methotrexate and pyrimethamine. Folinic acid is well-known by the skilled person. Any source of folinic acid may be used in the present invention. Folinic acid may also be used in combination with 5-fluorouracil to treat colorectal cancer. In this case, folinic acid enhances the effect of 5-fluorouracil by inhibiting thymidylate synthase. Further, folinic acid acts or contributes to protect against bone marrow suppression or gastrointestinal mucosa inflammation during chemotherapy. Folinic acid may be taken by mouth, injection into a muscle, or injection into a vein. Folinic acid has the chemical formula $C_{20}H_{23}N_7O_7$ and the following chemical structure (schematic D) (ChEMBL: CHEMBL1679; DRUGBANK accession number DB00650 / APRD00698):

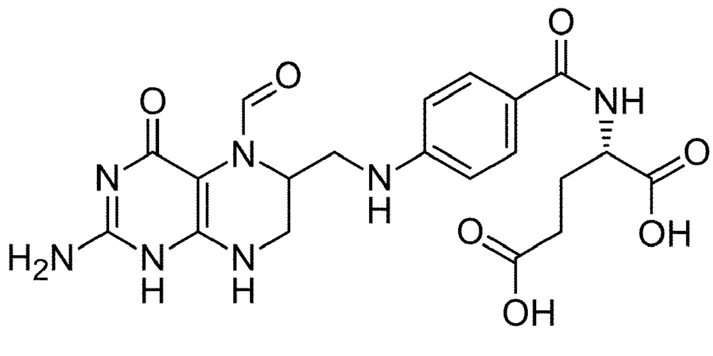

**Schematic D**. Folinic acid chemical structure

The term "FOLFOX" as used herein refers to a chemotherapy regimen for treatment of colorectal cancer, wherein the following drugs are combined: Folinic acid (leucovorin or FOL) and Fluorouracil (5-FU or F) and Oxaliplatin (Eloxatin or OX).

The term "CAPOX" (also called XELOX) as used herein refers to a chemotherapy regimen wherein the following drugs are combined: Capecitabine (abbreviated CAP; trade name Xeloda) and Oxaliplatin (Eloxatin or OX).

The term "CAPIRI" (also known as XELIRI) as used herein refers to a chemotherapy regimen wherein the following drugs are combined: capecitabine (abbreviated CAP; trade name Xeloda) and Irinotecan (abbreviated IRI, brand name Camptosar).

The term "FOLFIRI" as used herein refers to a chemotherapy regimen wherein the following drugs are combined: Folinic acid (leucovorin or FOL) and 5-fluorouracil (5-FU or F) and Irinotecan (abbreviated IRI or FI, brand name Camptosar).

The term "FI" as used herein refers to a chemotherapy regimen wherein the following drugs are combined: 5-fluorouracil (5-FU or F) and Irinotecan.

The term "FO" as used herein refers to a chemotherapy regimen wherein the following drugs are combined: 5-fluorouracil (5-FU or F) and Oxaliplatin.

It is understood that for FOLFOX, CAPOX, CAPIRI, FOLFIRI, FI and FO, the term "combined" means that the drugs may be provided together (e.g. in one composition or pill) at the same time, separately but substantially at the same time or subsequently (one immediately after the other, either via the same route of administration (e.g. orally) or via difference administration route (e.g. one drug is given orally, the other is injected (iv)), etc.

The term "non-responder" (or resistant or no clinical benefit) as used herein refers to a cancer patient (e.g. colorectal cancer patient) showing resistance to treatment or showing no meaningful clinical response to a given drug treatment, for instance the drug treatment according to the present invention (i.e.1) 5-FU or capecitabine in combination with irinotecan or 2) irinotecan alone) as evidenced by no reduction in tumor burden or size or volume, no significant improvement health status or deterioration in health status (e.g. due to toxicity), drug intolerance (e.g. due to toxicity). For instance, a gastrointestinal cancer patient may be identified as a non-responder by for instance showing progressive disease after 3 cycles of chemotherapy (or 2 months of treatment) according to RECIST 1.1 in said patient (Eisenhauer, E. A. et al (2009), Eur. J. Cancer, Vol. 45, pages 228-247). The skilled person knows how to determine whether a cancer patient is a "non-responder" to a given drug treatment.

The term "responder" (sensitive or clinical benefit) as used herein refers to a cancer patient (e.g. colorectal cancer patient) showing sensitivity to treatment or showing a meaningful clinical response to a given drug treatment, for instance the drug treatment according to the present invention (i.e.1) 5-FU or capecitabine in combination with irinotecan or 2) irinotecan alone) as evidenced by reduction in tumor burden or size or volume, improvement health status in or no deterioration in health status (e.g. no or minimal toxicity). For instance, a gastrointestinal cancer patient may be identified as a responder by for instance showing disease remission or partial disease remission or stable disease after 3 cycles of chemotherapy (or 2 months of treatment) according to RECIST 1.1 in said patient (Eisenhauer, E. A. et al (2009), Eur. J. Cancer, Vol. 45, pages 228-247). The skilled person knows how to determine whether a cancer patient is a "responder" to a given drug treatment.

The term "drug-induced growth rate inhibition" (abbreviated GR) as used herein refers to a metric that is used to measure or assess sensitivity of (dividing) cells such as cancer cells (or PDOs derived from a cancer sample) to (cancer) drugs. Traditional metrics used for the same purpose, such as IC50, suffer from a fundamental flaw when they are estimated from cell counts (e.g. number of cells alive) made at the end of the experiment (the standard approach), particularly when cells (e.g. cancer cells) undergo different numbers of divisions during the course of an assay due to natural differences in proliferation rate, variation in growth conditions, or changes in the duration of an experiment. Consequently, metrics such as IC50, E max, and AUC values may vary dramatically, independent of any changes in the underlying biology. This is undesirable as it may create experimental bias and provide inaccurate information about drug sensitivity. Away to overcome these limitations is to use GR metrics. A main advantage of using GR metrics in place of the traditional metrics such as IC50 is that GR metrics are insensitive to cell division and assay duration. Assessment of drug sensitivity using GR metrics is based on comparing growth rates in the presence and absence of drug. In the present invention, GR may be measured according to any suitable methods for assessing the growth rate of tumor cells in response to drug exposure. The GR (and methods for measuring GR) is described in details in for instance Hafner, M., et al (2016), Nat. Methods, Vol. 13, pages 521-527. Harris, L. A. et al (2016), Nat. Methods, Vol. 13, pages 497-500.

For instance, one such method for measuring or determining GR is as set out below, where GR is measured according to the following formula below, which is further described in Hafner, M., et al (2016), Nat. Methods, Vol. 13, pages 521-527. Harris, L. A. et al (2016), Nat. Methods, Vol. 13, pages 497-500:

$$GR(c) = 2^{\log_2(x(c)/x_0)/\log_2(x_{ctrl}/x_0)} - 1$$

wherein:

"GR(c): drug-induced growth rate inhibition
"x(c)" refers to the cell count in the presence of drug;
"Xctrl" refers to the 50%-trimmed mean of the cell count for control cells or mean of the cell count for control cells as derived from the luminescence assay.
"x0" is the 50%-trimmed mean of the cell count from a sample grown in parallel and measured just prior to drug exposure or the mean of the cell count from a sample grown in parallel and measured just prior to drug exposure.

The term "50%-trimmed mean" or "mean trimmed 50%" as used herein refers to a mean that is calculated by discarding a certain percentage of the lowest and the highest scores and then computing the mean of the remaining scores. For example, a mean trimmed 50% is computed by discarding the lower and higher 25% of the scores and taking the mean of the remaining scores. In the context of the invention, the use of the 50%-trimmed mean" or "mean trimmed 50%" is optional as one may use the non-trimmed mean (i.e. a mean that is calculated without discarding a certain percentage of the lowest and the highest scores) without affecting the reliability of the results obtained by the method.

It is understood that the term "cell count" means the number of cells alive (cell viability) after a given treatment (e.g. drug treatment consisting of 5-FU in combination with irinotecan) or no drug treatment (control situation), after a given duration or time point (e.g. before treatment or at the term of treatment, e.g. after 6 hours). Cell viability or number of cells alive can be assessed using any suitable methods in the art. For instance, cell viability may be assessed by using a luminescent assay such as CellTiter-Glo3D (Promega cat. Number G9681) and automated luminescence plate reader such as Infinite 200 Prop plate reader (Tecan Life Sciences). Other non-limiting methods suitable for assessing cell viability include 3-(4,5-dimethylthiazol-2-yl)-2,5-diphenyltetrazolium bromide (MTT) assay (e.g. see Grabinger, T. et al (2014), Cell Death and Disease, Vol. 5, e1228; doi:10.1038/cddis.2014.183) and LIVE/DEAD Viability Kit, Molecular Probes (see e.g. Pauli et al (2017), Cancer Discov, Vol: 7(5), pages 462-77).

The term "metastasis" or "cancer metastasis" as used herein refers to a situation where cancer cells break away from where they first formed (primary cancer), travel through the blood or lymph system, and form new tumors (metastatic tumors or lesions) in other (distal) parts of the body. For instance, gastrointestinal cancer, e.g. colorectal cancer or pancreatic cancer and others, is a type of cancer which often undergoes metastasis, i.e. spread to other regions of the body to form new cancer (also referred to as metastatic lesions) including for instance skin, lung lymph nodes, liver, breast, peritoneum, omentum, and others areas of the body.

## Methods for determining a reference value 1

In a first aspect, the present invention relates to an (in vitro) method for determining a reference value 1 (REF1) to segregate a first group of patients and a second group of patients, wherein the first group and second group of patients are composed of gastrointestinal cancer patients who are responders and non-responders, respectively, to a chemotherapeutic treatment comprising 5-fluorouracil or capecitabine in combination with irinotecan or irinotecan alone, said method comprising the step of:

**a.** Providing a tumor sample obtained from each gastrointestinal cancer patient in the first and second group of patients;
**b.** Generating a plurality of patient-derived tumor organoids (PDOs) from the tumor sample obtained from each gastrointestinal cancer patient in the first and second group of patients, thereby obtaining a PDO line for each gastrointestinal cancer patient in the first and second group of patients;
**c.** Contacting the PDO line of each gastrointestinal cancer patient in the first and second group of patients with 5-fluorouracil or capecitabine in combination with irinotecan or irinotecan alone at a first dosage, and optionally at a second or further dosage(s), for at least 3 days (preferably 6 days);
**d.** Calculating a drug-induced growth rate inhibition (GR) value for the PDO line of each gastrointestinal cancer patient in the first and second group of patients for the first dosage, and optionally for the second dosage or further dosage(s);
**e.** Calculating a GR score for the PDO line of each gastrointestinal cancer patient in the first and second group of patients, wherein the GR score is the GR value for the PDO line of each patient in the first and second group of patients for the first dosage or wherein the GR score is calculated by summing the GR value of the PDO line of each gastrointestinal cancer patient in the first and second group of patients for the first dosage and second dosage or further dosages, thereby obtaining a GR score for each gastrointestinal cancer patient in the first and second group of patients;
**f.** Establishing the REF1 value, wherein said REF1 is the highest GR score in the first group of patients that segregates a maximum number of gastrointestinal cancer patients in the first group of patients from a maximum of the gastrointestinal cancer patients in the second group of patients;

or

wherein said REF1 is the lowest GR score in the second group of patients that segregates a maximum number of gastrointestinal cancer patients in the second group of patients from a maximum of the gastrointestinal cancer patients in the first group of patients;

or

wherein said REF1 is a GR score that is between the highest GR score in the first group of patients and the lowest GR score in the second group of patients, wherein said GR score segregates a maximum number of gastrointestinal cancer patients in the first group of patients from a maximum of the gastrointestinal cancer patients in the second group of patients.

In step a) the tumor sample may be a biopsy from the primary tumor site orfrom a metastatic site. For instance, the tumor sample may be one or more piece(s) or slice(s) of tissue that has/have been removed from a tumor, including following a surgical tumor resection. The tumor tissue sample can be subjected to a variety of well-known post-collection preparative and storage techniques (e.g., enzymatic/mechanical dissociation, freezing etc.) In the present invention, the tumor tissue sample is a gastrointestinal cancer sample, e.g. where the gastrointestinal cancer may be selected from esophagus cancer, stomach cancer, biliary system cancer, small intestine cancer, large intestine cancer (or colon cancer), colorectal cancer, rectum cancer and anus cancer, preferably colorectal cancer. Further, the gastrointestinal cancer (tumor) sample may be taken from the primary tumor or from a metastatic lesion thereof (e.g. skin, lung, lymph node, liver, breast, peritoneum, or omentum, or others).

In the context of the present invention, it is understood that the REF1 value (or "threshold value" or "cut-off value") is a pre-determined value, i.e. information obtained from gastrointestinal cancer patients with a known clinical outcome (e.g. responders or non-responders) to a chemotherapeutic treatment comprising: 1) 5-fluorouracil or capecitabine in combination with irinotecan or 2) irinotecan alone. The REF1 value can be subsequently used in the method as taught herein for predicting responsiveness or treatment outcome of a gastrointestinal cancer patient to a chemotherapeutic treatment comprising: 1) 5-fluorouracil or capecitabine in combination with irinotecan or 2) irinotecan alone, using the method as taught below. It is understood that when using (running) the method for determining the reference value 1 (REF1) as taught herein for the purpose of predicting responsiveness of a gastrointestinal cancer patient to a chemotherapeutic treatment comprising 1) 5-FU or capecitabine in combination with irinotecan or 2) irinotecan alone (as taught herein), preferably the PDO's used in the method for determining REF1 and the PDOs used to determine responsiveness to a treatment are treated substantially the same way, i.e. substantially same experimental conditions such as same drug treatment, same dosage(s), same chemical reagents, same timeline, same drug exposure time, same analysis method (e.g. same cell viability assay), same way of calculating GR values and GR scores, etc.

It is further understood that, when determining the REF1 value according to the method as taught herein, any group of responders and group of non-responders (i.e. for which a priori information is available about treatment outcome chemotherapeutic treatments as taught herein) may be used without affecting the accuracy or reliability of the methods of the invention. In the context of the present invention, the term "group of responders" and "group of non-responders" mean a group of more than 2 responders or 2 non-responders, such as 2, 3, 4, 5, 6, 7, 8, 9, 10, 15, 20, 25, 30, 35, 40, 45, 50 or more responders or non-responders.

In step b), PDOs can be obtained using standard methods in the art such as for instance the method described in Wetering et al (2015), Cell, Vol: 161 (4), pages 933-945; Weeber et al (2015), PNAS, Vol: 112 (43), pages 13308-13311; Vlachogiannis, G., et al (2018), Science, Vol: 359, pages 920-926; and Tiriac, H., et al (2018), Cancer Discov., Vol:8(9); pages 1-18, doi:10.1158/2159-8290.CD-18-0349) or as described herein in the example section. It is understood that the term "a plurality of PDOs" refers to a quantity or amount of PDOs obtained in culture that is sufficient to reliably carry out the method as taught herein, e.g. to obtain reliable prediction about responsiveness to a given chemotherapeutic treatment such as 1) 5-FU or capecitabine in combination with irinotecan or 2) irinotecan alone. For instance when using a well-plate system and when testing several drugs and/or dosages of said drug(s), a sufficient amount of PDOs may be an amount that allows one to use about 5, 10, 20, 30, 40, 50, 60, 70, 80, 90, 100 PDOs or more per well, preferably about 100 PDOs per well, as this has been shown to reduce variation due to experimental settings or errors.

For instance, in order to have a sufficient amount of PDOs to generate about 100 PDOs per well for testing several dosages of one or more drugs, it may be advantageous to use a number of at least 3900 PDOs, such as 3400, 3500, 3600, 3700, 3800, 3900, 4000 or more PDOs. Such amount of PDOs can be obtained for instance from one biopsy (sample) of a primary or metastatic lesion, which is subsequently processed in culture for about 2-4 weeks according to standard protocols such as for instance the method described in Wetering et al (2015), Cell, Vol: 161 (4), pages 933-945; Weeber et al (2015), PNAS, Vol: 112 (43), pages 13308-13311; Vlachogiannis, G., et al (2018), Science, Vol: 359, pages 920-926; and Tiriac, H., et al (2018), Cancer Discov., Vol:8(9); pages 1-18, doi:10.1158/2159-8290.CD-18-0349) or as described herein in the example section. It is understood that depending on the nature of the experiments to be performed, e.g. how many drugs to be tested, dosages, replicate per dosage, number of patients, etc., the skilled person knows how to determine a suitable amount of PDOs (derived from one gastrointestinal cancer patient) for reliably carrying out the method of the invention. It is also understood that the numbers provided above are strictly exemplary, and are not limiting in any ways.

In step c), the term "contacting" refers to adding one or more compound(s) (e.g. 5-FU or capecitabine in combination with irinotecan or 2) irinotecan alone) to the culture medium in which the PDOs are incubated or exposing PDOs to a culture medium comprising one or more compound(s) (e.g. 1) 5-FU or capecitabine in combination with irinotecan or 2) irinotecan alone) for a given period of time. It is understood that the term "dosage" (or "dose") of a given drug compound refers to a measured amount (e.g. nanoM, microM) of said drug compound. The term dosage may also refers to a concentration of said drug compound, i.e. the abundance of said drug compound divided by the total volume of a mixture (e.g. culture medium) (e.g. microgram/ml). In the method of the present invention, each drug compound e.g. 5-FU , capecitabine, irinotecan) may be tested according to one dosage, preferably 2 different dosages, 3 different dosages, 4 different dosages, 5 different dosages or more. It may be advantageous to test a given drug compound at more than one dosage to improve the reliability of the methods as taught herein.

In step d) calculating the GR values of the PDO line of each gastrointestinal cancer patient may be determined using the formula as taught herein. For instance, the GR value for the PDO line of a gastrointestinal cancer patient may be calculated as follows:

**Gastrointestinal cancer patient 1 (P1) (e.g. colorectal cancer patient)**

A biopsy is taken from the primary tumor or from a metastatic lesion thereof (e.g. skin) of said patient. The biopsy is processed and expanded in culture to generate a (sufficient amount of PDOs) plurality of PDO's, e.g. around 4000 PDOs over a period of about two weeks, according to standard methods (e.g. Wetering et al (2015) Cell, Vol: 161 (4), pages 933-945; Weeber et al (2015), PNAS, Vol:112 (43), pages 13308-13311; Vlachogiannis, G., et al (2018), Science, Vol: 359, pages 920-926; and Tiriac, H., et al (2018), Cancer Discov., Vol:8(9); pages 1-18, doi:10.1158/2159-8290.CD-18-0349) or as described herein in the example section). In the context of the present invention, collection of sample, expansion of PDOs and drug exposures can be done rather quickly, for example in a period of 2 to 4 weeks.

**Well-plate:** The plurality of PDO's obtained from one given gastrointestinal cancer patient is divided into subgroups into a well-plate, wherein there is about 5, 10, 20, 30, 40, 50, 60, 70, 80, 90, 100 or more PDO's from said patient, preferably around 100 PDO's from said patient per well. In a well plate, there may be one or more wells containing a plurality of PDO's (e.g. 100 PDOs) from one given gastrointestinal cancer patient and there may be one or more wells containing a plurality of PDO's (e.g. 100 PDOs) from another gastrointestinal cancer patient, and so on. The skilled person understands that this will depend on the particular experimental set up. It is understood that each well comprising PDOs will be exposed to one particular drug treatment for a given amount of time, depending on the experimental set up.

**Drug treatment:** Separate wells with PDOs (1 PDO line/well) will be exposed to one of the following treatments, where each treatment is preferably performed in duplicate or more preferably in triplicate.

- Treatment 1: 5-FU in combination with irinotecan (alternatively it can also be capecitabine in combination with irinotecan).
  or
- Treatment 2: irinotecan alone. In the context of *in vitro* testing, the skilled person understands that SN-38, which is the active metabolite of irinotecan, can be used instead of irinotecan.
  or
  Treatment 3: 5-FU only
  or
- Treatment 4: No drug (control)

**Dosage (concentration):** Each drug treatment (i.e. treatment 1 and treatment 2) can be carried out wherein each drug compound(s) is tested in one or more dosages (i.e. concentration). For instance, two dosages (concentrations) may be tested per drug compound such as dosage a and dosage b.

**Time points:** For each treatment at a given dosage (concentration), cell viability (cell count) is measured at the following time points (e.g. by using a luminescent cell viability assay as descried above)

- Before treatment initiation: Time 0 (baseline)
- At the term of treatment: For instance, after 6 days (Time 6 days) of treatment 1 (5-FU and irinotecan).

The cell viability data are used to calculate the GR value of each PDO line (which corresponds to one particular gastrointestinal cancer patient) using the formula disclosed above for each drug treatment, for each dosage (concentration).

In the context of the present invention, if the GR value is above zero (between zero and 1), it indicates a certain degree of growth of the PDOs in response to the drug treatment, i.e. the PDOs/cancer cells continue to growth in the

presence of the therapeutic drug(s) (e.g. 5-fluorouracil or capecitabine in combination with irinotecan or irinotecan alone). In other words, it indicates that the number of cells remaining is greater than the number of cells initially plated at day 0. This may indicate that in general, the PDOs/ cancer cells are not responsive or minimally responsive to the drug treatment, at the dosage tested. Alternatively, if the GR value is equal or below zero, it indicates decreased growth (cell death) or no growth of the PDOs/cancer cells in response to the drug treatment, i.e. the PDOs/cancer cells stop growing or do not grow as much in the presence of the therapeutic drug(s) (e.g. 5-fluorouracil or capecitabine in combination with irinotecan or irinotecan alone). This indicates that in general, the PDOs/ cancer cells may be responsive to the drug treatment or that the PDOs/ cancer cells may be stable (stable disease, no progression), i.e. do not growth further. In other words, a GR value of 1 indicates no effect of the drug on growth (growth is the same as the growth of untreated cells). A GR value between 0 and 1 indicates a certain degree of growth but not cell death. A GR value of 0 indicates an effect of the drug on growth (the number of cells at the end of the treatment is the same as the number of cells on day 0, which indicates no growth at all). A GR value between 0 and -1 indicates an effect of the drug on growth (it indicates cell death).

The same procedure can be repeated for one or more subsequent gastrointestinal cancer patients (e.g. 2, 5, 10, 20, 50 or more patients). The experimental set up for treatment 1 is summarized in schematic 1 below for 2 colorectal cancer patients (P1 and P2):

| Patient ID | Replicate 1 | Replicate 2 | Replicate 3 |
|---|---|---|---|
| P1 (dosage a) | GR1a = 0.35 | GR1a = 0.33 | GR1a = 0.40 |
| P1 (dosage b) | GR1b = 0.42 | GR1b = 0.45 | GR1b =0.33 |
| P2 (dosage a) | GR2a = -0.30 | GR2a = -0.28 | GR2a = -0.25 |
| P2 (dosage b) | GR2b = -0.50 | GR2b = -0.48 | GR2b =-0. 37 |

**Schematic 1.** Representative experimental set up for treatment 1 (5-Fu and irinotecan) in a well-plate system, wherein each well comprises about 100 PDOs. P1= patient 1, P2 = patient 2, dosage a (5-Fu at a dosage of 0.5 microM and irinotecan at a dosage of 0.25 microM), dosage b ((5-Fu at a dosage of 200 microM and irinotecan at a dosage of 100 microM), GR1a= drug-induced growth inhibition for dosage a for patient 1 (in triplicate), GR1b= drug-induced growth inhibition for dosage b for patient 1 (in triplicate), GR2a= drug-induced growth inhibition for dosage a for patient 2 (in triplicate), and GR2b= drug-induced growth inhibition for dosage b for patient 2 (in triplicate).

It is understood that the numbers provided above are strictly exemplary, and are not limiting in any ways.

In step e), the GR score of the PDO line for each gastrointestinal cancer patient may be determined or calculated as follows

**Patient 1 (P1):** GR score of the PDO line of each gastrointestinal cancer patients are determined by summing the median values or average GR1a values (e.g. average: 0.35 + 0.33 + 0.40 divided by 3 = 0.36, data taken from the table above) and average GR1b values (0.42 + 0.45 + 0.33 divided by 3 = 0.40, data taken from the table above). The summation of average GR1a + average GR1b = 0.36 + 0.40 = **0.76**. Therefore patient 1 has a GR score of **0.76**.

**Patient 2 (P2):** GR score are determined by summing the median values or average GR2a values (i.e. -0.30 + -0.28 + -0.25 divided by 3 = -0.27, data taken from the table above) and average GR2b values (i.e. -0.50 + -0.48 + -0.37 divided by 3 = 0.45, data taken from the table above). The summation of average GR2a + average GR2b = -0.27 + -0.45 = **-0.72**. Therefore patient 2 has a GR score of **-0.72**.

The meaning of the GR value is as above.

In the context of the present invention, the mean (or arithmetic mean) is defined as the sum of all the numbers in the set divided by the amount of numbers in the set while the median is the middle point of a number set, in which half the numbers are above the median and half are below.

It is understood that the numbers provided above are strictly exemplary, and are not limiting in any ways.

In step f) determining reference value 1 (REF1) may be determined as follows:
In order to stratify or segregate or identify patients (based on PDO line derived thereof) as responders or non-responder to a given drug treatment, such as treatment with: 1) 5-FU or capecitabine in combination with irinotecan) or 2) (irinotecan), a reference value 1 (REF1) is first determined (pre-determined) as shown below.

**Step 1a:** Biopsies are taken from a group of gastrointestinal cancer patients (e.g. n=6), which are known (*a priori*) as *responder* to a given drug treatment, such as treatment 1 (5-Fu or capecitabine in combination with irinotecan) or treatment 2 (irinotecan).

**Step 1b:** Biopsies are taken from a group of gastrointestinal cancer patients (e.g. n=6), which are known (*a priori*) as *non-responder* to a given drug treatment, such as treatment 1 (5-FU or capecitabine in combination with irinotecan) or treatment 2 (irinotecan).

The procedure above for generating PDOs (1 PDO line per patient), calculate GR values and GR scores is repeated for each gastrointestinal cancer patient in the responder group and for each gastrointestinal cancer patient in the non-responder group, per treatment (e.g. 5-FU in combination with irinotecan).

The GR score for each patient in each group are then studied or plotted. A cut-off line is then fitted so that the line divides the maximum amount of responders from the maximum amount of non-responders, as best as possible. In order to achieve that, the REF1 value may be selected as follows:

- ➢ Option 1: The REF 1 is the highest GR score in the group of responders that segregates (divides) a maximum number of responders from a maximum of non-responders (;
or
- ➢ Option 2: The REF 1 is the lowest GR score in the group of non-responders that segregates a maximum number of non-responders from a maximum of responders;
or
- ➢ Option 3: The REF1 is a GR score that is located anywhere between the highest GR score in the group of responders and the lowest GR score in the group of non-responders, wherein said GR score segregates a maximum number of responders from a maximum of the non-responders.

In a preferred embodiment, the REF 1 is according to option 1.

The skilled person understands that in certain embodiments, the REF1 will be chosen depending on what patient group needs to be identified, i.e. responders or non-responders or depending on whether higher specificity or sensitivity is desired for identifying responders or depending on whether higher specificity or sensitivity is desired for identifying non-responders.

<u>Method for predicting treatment outcome and for selecting patients</u>

In a further aspect, the present invention relates to an (in vitro) method for predicting responsiveness or treatment outcome of a gastrointestinal cancer patient to a chemotherapeutic treatment comprising 5-fluorouracil or capecitabine in combination with irinotecan or irinotecan alone, said method comprising the steps of:

a. Providing a plurality of patient-derived tumor organoids (PDOs) obtained from said gastrointestinal cancer patient, thereby obtaining a PDO line for said gastrointestinal cancer patient;
b. Contacting the PDO line of the gastrointestinal cancer patient with 5-fluorouracil or capecitabine in combination with irinotecan or irinotecan alone at a first dosage and optionally at second dosage or further dosage (s) for at least 3 days;
c. Calculating a drug-induced growth rate inhibition (GR) value for the PDO line of said gastrointestinal cancer patient for the first dosage, and optionally for the second dosage or further dosage(s);
d. Calculating a GR score for the PDO line of said gastrointestinal cancer patient, wherein the GR score is the GR value for the PDO line of said patient for the first dosage or wherein the GR score is calculated by summing the GR value of the PDO line of said gastrointestinal cancer patient for the first dosage and second dosage or further dosages, thereby obtaining a GR score for said gastrointestinal cancer patient; and
e. Predicting responsiveness or treatment outcome of the gastrointestinal cancer patient to the chemotherapeutic treatment comprising 5-fluorouracil or capecitabine in combination with irinotecan or irinotecan alone, wherein: i) if the GR score of the PDO line for said gastrointestinal cancer patient is equal or greater than a reference 1 (REF1) value, than it indicates that said gastrointestinal cancer patient is a non-responder or is less likely to respond to the chemotherapeutic treatment comprising 5-fluorouracil or capecitabine in combination with irinotecan or irinotecan alone; or ii) if the GR score of the PDO line for said gastrointestinal cancer patient is lower than a REF1 value, than it indicates that said gastrointestinal cancer patient is a responder or is more likely to respond to the chemotherapeutic treatment comprising 5-fluorouracil or capecitabine in combination with irinotecan or irinotecan alone; wherein the REF 1 value is determined according to the method of claim 1;
f. optionally, repeating steps (a) to (e) for subsequent PDO lines from said gastrointestinal cancer patient

It is understood that the method as taught herein can be used for accurate / reliable prediction of therapy / treatment outcome (predicting responsiveness or resistance to a given drug treatment) or to accurately select or identify a cancer subject (e.g. colorectal cancer patient) who is suitable (or not) for therapy with: 1) 5-fluorouracil or capecitabine in combination with irinotecan or 2) irinotecan alone.

Steps a) to d) may be performed as taught above.

In step e) the term "predicting responsiveness (or treatment outcome) of a gastrointestinal cancer patient to

the chemotherapeutic treatment comprising: 1) 5-fluorouracil or capecitabine in combination with irinotecan or 2) irinotecan alone, based on the GR score of the PDO line of said gastrointestinal cancer patient, means that depending on the GR score of the PDO line for said gastrointestinal cancer patient (and using the REF1 value as a cutoff value), one can accurately predict or identify whether said gastrointestinal cancer patient (from which the PDO line is derived) will respond (i.e. is a responder) or will not respond (i.e. is a non-responder) to treatment with: 1) 5-FU or capecitabine in combination with irinotecan or 2) irinotecan alone, prior the said treatment is initiated; or whether said gastrointestinal cancer patient will be suitable (i.e. is a responder) or will not be suitable (i.e. is a non-responder) for treatment with: 1) 5-FU or capecitabine in combination with irinotecan or 2) irinotecan alone, prior the said treatment is initiated. For instance, if the REF 1 value (as determined by the method as taught herein) is for instance a GR score of 0.5, than a PDO line derived from a gastrointestinal cancer patient having a GR score of for instance 0.4 or 0.3, or 0.2, or 0.1 or 0 or -1.0, or -1.5, or -2.0, or lower indicates or predicts that said gastrointestinal cancer patient is a responder or is more likely to respond to the chemotherapeutic treatment comprising 5-fluorouracil or capecitabine in combination with irinotecan or irinotecan alone.

Or

If the if the REF 1 value (as determined by the method as taught herein) is for instance a GR score of 0.5, than a PDO line derived from a gastrointestinal cancer patient having a GR score of for instance 0.5 indicates or predicts that said gastrointestinal cancer patient is a non-responder or is less likely to respond to the chemotherapeutic treatment comprising 5-fluorouracil or capecitabine in combination with irinotecan or irinotecan alone.

Or

If the if the REF 1 value (as determined by the method as taught herein) is for instance a GR score of 0.5, than a PDO line derived from a gastrointestinal cancer patient having a GR score of for instance 0.55, 0.60, 0.70, 0.80, 0.90, 1.0, or more, indicates or predicts that said gastrointestinal cancer patient is a non-responder or is less likely to respond to the chemotherapeutic treatment comprising 5-fluorouracil or capecitabine in combination with irinotecan or irinotecan alone.

In an embodiment, the in vitro method for predicting responsiveness (or treatment outcome) of a particular gastrointestinal cancer patient to the chemotherapeutic treatment comprising: 1) 5-fluorouracil or capecitabine in combination with irinotecan or 2) irinotecan alone (as taught herein) can be repeated (e.g. by taking a further tumor sample to generate a PDO line and run the method as taught above) one or more times (e.g. 2, 5, 10, 20, 30, 40, 50, 60, 70, 80, 90, 100 or more times) for said one particular patient. This may be advantageous to increase the reliability of the method, i.e. more accurate prediction of responsiveness to the chemotherapeutic treatment for said patient or to avoid false positive or false negative results.

As taught herein and as further demonstrated in the example section, the present inventors have found that the methods of the present invention can be reliably used (e.g. with high predicting power) to predict the responsiveness (or treatment outcome) of a gastrointestinal cancer patient (e.g. colorectal cancer or pancreatic cancer patient or others) to a chemotherapeutic treatment (prior onset of treatment) comprising: 1) 5-fluorouracil or capecitabine in combination with irinotecan or 2) irinotecan alone, prior initiating the treatment and without the need for a priori information on the gastrointestinal cancer patient. These findings are surprising and unexpected because responsiveness of a gastrointestinal cancer patient (e.g. colorectal cancer patient or pancreatic cancer patient or others) to another standard of care chemotherapeutic treatment comprising 5-FU and oxaliplatin could not be predicted using the method of the invention (i.e. GR values/scores of the PDO line and REF1 values derived thereof did not allow stratifying or identifying gastrointestinal cancer patients into responders and non-responders, as shown herein in the example section.

The present inventors further found a positive correlation between in vitro response of PDOs to a chemotherapeutic treatment comprising 5-FU or capecitabine in combination with irinotecan, or to a chemotherapeutic treatment comprising irinotecan alone, and in vivo response to the same treatment in patients. Such correlation was absent (not observed) in response to treatment 5-FU and oxaliplatin. Taken together, these results demonstrate the unique predictive power of the method of the invention in the context of chemotherapy with 5-FU or capecitabine in combination with irinotecan or with irinotecan alone.

The method of the invention provides a new and reliable predictive assay for chemotherapeutic treatment for gastrointestinal cancer patients, which can be advantageously used to decide on or assign the best treatment option(s) to a gastrointestinal cancer patient (particularly colorectal cancer patients or pancreatic cancer patients), to prevent exposing said patient to the unnecessary side effects associated with a standard of care chemotherapeutic treatment which will prove to be ineffective at a later stage (e.g. because it turns out the patient is a non-responder), and/or to reduce the financial burden associated with ineffective cancer treatment options. Other benefits of the present invention will become obvious throughout the embodiments as taught herein.

In an embodiment relating to the methods as taught herein the gastrointestinal cancer patient suffers from a gastrointestinal cancer that may be a metastatic gastrointestinal cancer (spread outside the primary locus) or may be a primary gastrointestinal cancer (primary locus/site where the cancer initially developed), preferably a metastatic gastrointestinal cancer.

In an embodiment relating to the methods as taught herein, the gastrointestinal cancer patient suffers from a gastrointestinal cancer that may be selected from esophagus cancer, stomach cancer, biliary system cancer, small

intestine cancer, large intestine cancer (or colon cancer), colorectal cancer, rectum cancer, anus cancer, and pancreatic cancer.

In a preferred embodiment relating to the methods as taught herein, the gastrointestinal cancer is colorectal cancer (CRC) or pancreatic cancer, at any stage of the disease.

In an embodiment relating to the methods as taught herein, the gastrointestinal cancer patient is preferably naive to the chemotherapy treatment comprising 5-fluorouracil or capecitabine in combination with irinotecan or irinotecan alone. In other words, this means that the gastrointestinal cancer patient has not been treated before with a chemotherapy treatment comprising 5-fluorouracil or capecitabine in combination with irinotecan or irinotecan alone.

In embodiment relating to the method as taught herein, the PDO line for each gastrointestinal cancer patient may be contacted with 1) 5-fluorouracil or capecitabine in combination with irinotecan or 2) irinotecan alone for a period of time ranging from 3 days to 15 days, or for 3, 4, 5, 6, 7, 8, 9, 10, 11, 12, 13, 14 or 15 days.

In a preferred embodiment relating to the methods as taught herein, the PDO line for each gastrointestinal cancer patient may be contacted with: 1) 5-fluorouracil or capecitabine in combination with irinotecan or 2) irinotecan alone for a period of at least 6 days, such as 6, 7, 8, 9, 10, 11, 12, 13, 14 or 15 days, preferably 6 days. In an embodiment, a period of at least 6 hours may be particularly advantageous because it allows sufficient time to reliably detect the effects of the therapeutic treatment on GR of the PDOs obtained from a given gastrointestinal cancer patient while being a length of time that is not too long for said gastrointestinal cancer patient, who is urgently awaiting for a suitable treatment.

In embodiment relating to the method as taught herein, the PDO line for each gastrointestinal cancer patient may be derived from a primary tumor or a metastatic lesion within each gastrointestinal cancer patient. It may be advantageous to generate the plurality of PDOs (PDO line) from a metastatic lesion because (in general) metastatic lesions are more easily accessible than primary tumors. It is understood that the predictive value or power of the methods as taught herein remain high (does not vary) irrespective of whether the plurality of PDOs (PDO lines) are derived from a primary tumor or a metastatic lesion.

In embodiment relating to the method as taught herein, the metastatic lesion may be taken from a region in the body of each gastrointestinal cancer patient that is selected from skin, lung, lymph node, liver, breast, peritoneum, and omentum. It is understood that the predictive value or power of the methods as taught herein remain high (does not vary) irrespective of whether the metastatic lesion is taken from the skin, lung, lymph node, liver, breast, peritoneum, or omentum.

In an embodiment relating to the method as taught herein, the chemotherapeutic treatment may further comprises one or more therapeutic compounds, and thus contacting the PDO line for each gastrointestinal cancer patient may further comprises one or more therapeutic compounds.

The term "therapeutic compound(s) or agent(s)" as used herein refers to an active compound(s) or drug(s) used in the treatment of a disease, such as gastrointestinal cancer (e.g. colorectal cancer)). In the context of the present invention, the one or more therapeutic compounds may be any suitable therapeutic compounds, e.g. that is compatible with 1) 5-FU or capecitabine in combination with irinotecan or 2) irinotecan alone.

In a preferred embodiment, the therapeutic compounds may be folinic acid and/or bevacizumab. In a further preferred embodiment, the therapeutic compounds is folinic acid.

It is understood that the therapeutic compound(s) or agent(s)" does not affect the predictive value or power of the methods as taught herein.

In an embodiment relating to the methods as taught herein, the PDO line of each gastrointestinal cancer patient may be contacted with 5-fluorouracil or capecitabine in combination with irinotecan, simultaneously or sequentially. It is understood that this may be done via the same route of administration (e.g. orally) or via different route of administration (e.g. one drug may be provided orally while the other may be provided intravenously (iv)), etc. For instance, 5-fluorouracil or capecitabine may be first added to the culture medium containing the PDOs (PDO line), followed by irinotecan being immediately added after or in a short period of time after (e.g. within 1, 2, 3, 4, or 5 minutes) to the same culture medium containing the same PDOs (PDO line). Alternatively, 5-fluorouracil or capecitabine may be added to the culture medium containing the PDOs (PDO line) substantially at the same time as irinotecan is being added to the same culture medium containing the same PDOs (PDO line).

## Medical uses

In a further embodiment, the present invention relates to the 5-fluorouracil or capecitabine in combination with irinotecan or irinotecan alone for use in a method for treating a gastrointestinal cancer patient, said method comprising predicting responsiveness or treatment outcome of a gastrointestinal cancer patient to a chemotherapeutic treatment comprising 5-fluorouracil or capecitabine in combination with irinotecan or irinotecan alone, said method comprising the steps of:

a. Providing a plurality of patient-derived tumor organoids (PDOs) obtained from said gastrointestinal cancer patient,

thereby obtaining a PDO line for said gastrointestinal cancer patient;

b. Contacting the PDO line of the gastrointestinal cancer patient with 5-fluorouracil or capecitabine in combination with irinotecan or irinotecan alone at a first dosage and optionally at second dosage or further dosage (s) for at least 3 days;

c. Calculating a drug-induced growth rate inhibition (GR) value for the PDO line of said gastrointestinal cancer patient for the first dosage, and optionally for the second dosage or further dosage(s);

d. Calculating a GR score for the PDO line of said gastrointestinal cancer patient, wherein the GR score is the GR value for the PDO line of said patient for the first dosage or wherein the GR score is calculated by summing the GR value of the PDO line of said gastrointestinal cancer patient for the first dosage and second dosage or further dosages, thereby obtaining a GR score for said gastrointestinal cancer patient; and

e. Predicting responsiveness or treatment outcome of the gastrointestinal cancer patient to the chemotherapeutic treatment comprising 5-fluorouracil or capecitabine in combination with irinotecan or irinotecan alone, wherein: i) if the GR score of the PDO line for said gastrointestinal cancer patient is equal or greater than a reference 1 (REF1) value, than it indicates that said gastrointestinal cancer patient is a non-responder or is less likely to respond to the chemotherapeutic treatment comprising 5-fluorouracil or capecitabine in combination with irinotecan or irinotecan alone; or ii) if the GR score of the PDO line for said gastrointestinal cancer patient is lower than a REF1 value, than it indicates that said gastrointestinal cancer patient is a responder or is more likely to respond to the chemotherapeutic treatment comprising 5-fluorouracil or capecitabine in combination with irinotecan or irinotecan alone; wherein the REF 1 value is determined according to the method of claim 1.

f. optionally, treating the gastrointestinal cancer patient of step i with a treatment that does not comprise 5-fluorouracil or capecitabine in combination with irinotecan or irinotecan alone;

g. optionally, treating the gastrointestinal patient of step ii with a chemotherapeutic treatment comprising 5-fluorouracil or capecitabine in combination with irinotecan or irinotecan alone;

h. optionally, repeating steps (a) to (g) for subsequent PDO lines from said gastrointestinal cancer patient.

The parameters in steps (a) to (h) may be determined or selected as taught above. The advantages as taught herein also fully apply here.

Method of treatment

In a further aspect, the present invention relates to a method of treating a gastrointestinal cancer patient, said method comprising predicting responsiveness or treatment outcome of a gastrointestinal cancer patient to a chemotherapeutic treatment comprising 5-fluorouracil or capecitabine in combination with irinotecan or irinotecan alone, said method comprising the steps of:

a. Providing a plurality of patient-derived tumor organoids (PDOs) obtained from said gastrointestinal cancer patient, thereby obtaining a PDO line for said gastrointestinal cancer patient;

b. Contacting the PDO line of the gastrointestinal cancer patient with 5-fluorouracil or capecitabine in combination with irinotecan or irinotecan alone at a first dosage and optionally at second dosage or further dosage (s) for at least 3 days;

c. Calculating a drug-induced growth rate inhibition (GR) value for the PDO line of said gastrointestinal cancer patient for the first dosage, and optionally for the second dosage or further dosage(s);

d. Calculating a GR score for the PDO line of said gastrointestinal cancer patient, wherein the GR score is the GR value for the PDO line of said patient for the first dosage or wherein the GR score is calculated by summing the GR value of the PDO line of said gastrointestinal cancer patient for the first dosage and second dosage or further dosages, thereby obtaining a GR score for said gastrointestinal cancer patient; and

e. Predicting responsiveness or treatment outcome of the gastrointestinal cancer patient to the chemotherapeutic treatment comprising 5-fluorouracil or capecitabine in combination with irinotecan or irinotecan alone, wherein: i) if the GR score of the PDO line for said gastrointestinal cancer patient is equal or greater than a reference 1 (REF1) value, than it indicates that said gastrointestinal cancer patient is a non-responder or is less likely to respond to the chemotherapeutic treatment comprising 5-fluorouracil or capecitabine in combination with irinotecan or irinotecan alone; or ii) if the GR score of the PDO line for said gastrointestinal cancer patient is lower than a REF1 value, than it indicates that said gastrointestinal cancer patient is a responder or is more likely to respond to the chemotherapeutic treatment comprising 5-fluorouracil or capecitabine in combination with irinotecan or irinotecan alone; wherein the REF 1 value is determined according to the method of claim 1.

f. optionally, treating the gastrointestinal patient of step i with a treatment that does not comprise 5-fluorouracil or capecitabine in combination with irinotecan or irinotecan alone;

g. optionally, treating the gastrointestinal patient of step ii with a chemotherapeutic treatment comprising 5-fluorouracil

or capecitabine in combination with irinotecan or irinotecan alone;
h. optionally, repeating steps (a) to (g) for subsequent PDO lines from said gastrointestinal cancer patient.

The parameters in steps (a) to (h) may be determined or selected as taught above. The advantages as taught herein also fully apply here.

It is contemplated that any method, use or composition described herein can be implemented with respect to any other method, use or composition described herein. Embodiments discussed in the context of methods, use and/or compositions of the invention may be employed with respect to any other method, use or composition described herein. Thus, an embodiment pertaining to one method, use or composition may be applied to other methods, uses and compositions of the invention as well.

It will be understood that all details, embodiments and preferences discussed with respect to one aspect of embodiment of the invention is likewise applicable to any other aspect or embodiment of the invention and that there is therefore not need to detail all such details, embodiments and preferences for all aspect separately.

Having now generally described the invention, the same will be more readily understood through reference to the following examples which is provided by way of illustration and is not intended to be limiting of the present invention. Further aspects and embodiments will be apparent to those skilled in the art.

## BRIEF DESCRIPTION OF THE DRAWINGS

Embodiments of the invention are further described hereinafter with reference to the accompanying drawings, in which:

**Figure 1.** Establishment of patient-derived organoids from tumors of patients in the TUMOROID trial. **Panel A** depicts a flow chart indicating the number of patients with metastatic CRC included, the number of evaluable patients, reasons for non-evaluability, and the success rate of establishing cultures from patients. Venn diagram illustrates the number of pre- and post-treatment PDOs per treatment cohort. FO: 5-FU OR capecitabine combined with oxaliplatin; FI: 5-FU or capecitabine combined with irinotecan. **Panel B** depicts a pie chart showing the site of the biopsied metastatic lesions from which PDOs were derived. **Panel C** depicts a boxplot of the time to first and second passage, and the time to generate a master biobank containing approximately 2-3 million PDOs. The box represent the 2nd quartile, the median and the third quartile. Error bars indicate range.

**Figure 2.** PDO drug sensitivity predicts outcome to treatment with 5-FU or capecitabine and irinotecan. **Panel A** shows an overview of the cohort of PDO cultures treated with FI. **Panel B** shows a waterfall plot of the patient overall response and best response of the biopsied lesion in the FI-treated PDO cohort. * indicates progressive disease (PD) due to appearance of new lesions. **Panel C** shows representative results of the in vitro drug sensitivity screen. Top panels: Heatmaps representing the GR score for each of the 36 data points in the combination drug sensitivity matrix. P22 is a patient sensitive to FI treatment, while P3.2 is a patient resistant to FI treatment. Bottom panels: Single-agent dose response curves for 5-FU (left panel) and SN-38 (right panel). Error bars represent the standard deviation of 2 independent experiments, each performed in technical triplicate. **Panel D** shows a correlation plot of screen 1 versus screen 2, performed by independent researchers. For each PDO, the GR value of each of the 11 data points in the 2 complementary dose response curves identified as having the largest variance within our samples was plotted, with the values from screen 1 on the x-axis and the values from screen 2 on the y-axis. **Panel E** shows GR scores of FI-treated organoids derived from tumors of patients with partial response (PR) / stable disease (SD) versus progressive disease (PD), based on lesion response. For each panel, scores of PR/SD (n=6) versus PD (n=6) patients were compared using the Mann-Whitney test. The screen was plated in technical triplicate and performed 2 times, once each by 2 independent researchers. This calculation was performed for both replicates of the screen, and the average of these 2 values is reported. In the left panel GR scores are graphed, representing in vitro sensitivity of PDOs to FI, calculated based on summing the 36 values of the data points in the full combination matrix. In the top 2 panels on the right, the log base 2 of the GR50 of either 5-FU (left panel) or SN-38 (right panel) are graphed. In the bottom 2 panels on the right, the AUC of either 5-FU (left panel) or SN-38 (right panel) are graphed. Dots/squares represent individual PDOs and error bars indicate standard error. **Panel F** shows GR scores of FI-treated PDOs derived from lesions with partial response (PR) / stable disease (SD) versus progressive disease (PD), based on lesion response. Scores of PR/SD (n=6) versus PD (n=6) patients were compared using a two-tailed Mann-Whitney test. The screen was plated in technical triplicate and performed 2 times, once each by 2 independent researchers. GR scores representing in vitro sensitivity of PDOs to FI were calculated by summing the 11 data points in the sub-matrix. This calculation was performed for both replicates of the screen, and the average of these 2 values is reported. Dots/squares represent individual PDOs and error bars indicate standard error. **Panel G** shows the ROC curve of the FI cohort illustrating the potential to predict lesion response (green line) and overall response

(blue line). auROC = area under the ROC curve, CI = confidence interval. **Panel H** shows a Kaplan-Meier curve depicting the PFS of sensitive (Z-score < 0.0) versus resistant (Z-score > 0.0) PDOs. Groups were compared using the Mantel-Cox log-rank test. **Panel I** shows a summary table of the area under the ROC curve (auROC) and 95% confidence interval (CI) for either lesion or overall response, calculated based on various in vitro parameters.

**Figure 3.** PDO drug sensitivity does not predict outcome to treatment with 5-FU/capecitabine and oxaliplatin. **Panel A** shows a waterfall plot of the patient overall and best lesion responses in the FI-treated PDO cohort. * indicates PD due to appearance of new lesions. **Panel B** shows an overview of the cohort of PDO cultures treated with FO. **Panel C** shows a correlation plot of screen 1 versus screen 2, performed by independent researchers. For each PDO, the GR value of each of the 36 data points in full combination matrix was plotted, with the values from screen 1 on the x-axis and the values from screen 2 on the y-axis. **Panel D** shows GR scores of FO-treated PDOs derived from lesions with partial response (PR) / stable disease (SD) versus progressive disease (PD), based on lesion response. Scores of PR/SD (n=13) versus PD (n=4) patients were compared using a two-tailed Mann-Whitney test. The screen was plated in technical triplicate and performed 2 times, once each by 2 independent researchers. GR scores representing in vitro sensitivity of PDOs to FO were calculated by summing the 6 data points in the 1:1 diagonal dose-response curve of 5-FU and oxaliplatin. This calculation was performed for both replicates of the screen, and the average of these 2 values is reported. Dots/squares represent individual PDOs (except for P1a-d, which are represented as the average of these 4 samples) and error bars indicate standard error. **Panel E** shows GR scores of FO-treated organoids derived from tumors of patients with partial response (PR) / stable disease (SD) versus progressive disease (PD), based on lesion response. For each panel, scores of PR/SD (n=13) versus PD (n=4) patients were compared using the Mann-Whitney test. The screen was plated in technical triplicate and performed 2 times, once each by 2 independent researchers. This calculation was performed for both replicates of the screen, and the average of these 2 values is reported. In the left panel, GR scores are graphed representing in vitro sensitivity of PDOs to FO, calculated based on summing the 36 values of the data points in the full combination matrix. In the top panels 2 on the right, the log base 2 of the GR50 of either 5-FU (left panel) or oxaliplatin (right panel) are graphed. In the bottom 2 panels on the right, the AUC of either 5-FU (left panel) or oxaliplatin (right panel) are graphed. Dots/squares represent individual PDOs (except for P1a-d, which are represented as the average of these 4 samples) and error bars indicate standard error. **Panel F** shows the ROC curve of the FO cohort illustrating the potential to predict lesion response (green line) and overall patient response (blue line). auROC = area under the ROC curve, CI = confidence interval. **Panel G** shows a summary table of the area under the ROC curve (auROC) and 95% confidence interval (CI) for either lesion or overall response, calculated based on various in vitro parameters.

**Figure 4.** Linear regression analysis to model the relationship between GR score and RECIST 1.1. RECIST 1.1 values were plotted against the sum of the GR values of the 11 data points in the sub-matrix. A linear regression was performed to identify the relationship between GR score and RECIST 1.1.

## EXAMPLES

Example 1: Predicting responsiveness to a chemotherapeutic treatment (drug treatment)

### Materials and methods

Study design

The TUMOROID study is a Dutch multi-centre observational cohort study (NL49002.031.14). The objective of the study is to evaluate the potential of PDOs to distinguish patients with, from patients without, response to standard-of-care treatment and the primary endpoint is a standardized PDO-based test with an area under the curve of the Receiver Operating Characteristic (auROC) of >0.7 and a high negative predictive value (e.g. the ability to exclude non-responder without withholding treatment to responders).

The study was approved by the ethical review board of The Netherlands Cancer Institute. The protocol complies with the Declaration of Helsinki, Dutch law, and Good Clinical Practice. All patients provided written informed consent prior to any study-related procedures. Patients with metastatic colorectal cancer (CRC) were accrued at the Netherlands Cancer Institute, Meander Medical Centre Amersfoort, and Elisabeth-TweeSteden Hospital Tilburg. Eligibility criteria included an Eastern Cooperative Oncology Group (ECOG) performance status of ≤2; measurable disease; histologic tumour biopsy feasible; and age of 18 years or older. Patients underwent pre-treatment biopsies before start of treatment with clinically approved regimens of capecitabine or fluorouracil (5-FU) combined with oxaliplatin or irinotecan (described below). Compulsory post-treatment biopsies were taken upon clinical progression. Patients underwent CT-scans at baseline and every 2 months to monitor response to treatment. Clinical outcome was score using RECIST 1.1 (Eisenhauer, E. A. et al (2009), Eur. J. Cancer, Vol. 45, pages 228-247).

Treatment

Patients were treated according to clinically approved combination regimens of capecitabine or 5-fluorouracil (5-FU) in combination with oxaliplatin or irinotecan. Bevacizumab (a recombinant humanized monoclonal antibody that blocks angiogenesis by inhibiting vascular endothelial growth factor A (VEGF-A)) was allowed in either combination.

CAPOX (capecitabine combined with oxaliplatin) was given in 3 week cycles; patients received capecitabine 1000 mg/m2 orally twice a day on day 1-14 and oxaliplatin 130 mg/m2 intravenously on day 1.
FOLFOX (folinic acid and fluorouracil (5-FU or F) and oxaliplatin) was given in cycles of 2 weeks; patients received oxaliplatin 85mg/m2, leucovorin 400 mg/m2, and fluorouracil (400 mg/m2 as bolus, 600 mg/m2 in 22 hours) intravenously on day 1. On day 2, the patient received leucovorin 200 mg/m2 and fluorouracil (400 mg/m2 as bolus, 600 mg/m2 in 22 hours) intravenously.

CAPIRI (capecitabine and irinotecan) was given in 3-week cycles, where patients received capecitabine 1000 mg/m2 orally twice a day on day 1-14 and irinotecan 250 mg/m2 intravenously on day 1.
FOLFIRI (folinic acid and 5-fluorouracil and irinotecan) was given in cycles of 2 weeks; patients received irinotecan 180 mg/m2, leucovorin 200 mg/m2, and fluorouracil (400 mg/m2 as bolus, 600 mg/m2 in 22 hours) intravenously on day 1. On day 2, the patient received leucovorin 200 mg/m2 and fluorouracil (400 mg/m2 as bolus, 600 mg/m2 in 22 hours) intravenously.

Patient material processing and organoid culture

Two to 4, 18 Gauge tumour biopsies were used for organoid culture and DNA-sequencing. Biopsies were collected in Advanced Dulbecco's Modified Eagles Medium with Nutrient Mixture F-12 Hams (Ad-DF) (Invitrogen; #12634), supplemented with 1% penicillin/streptomycin (Invitrogen; #15140-122), 1% HEPES (Invitrogen; #15630-056) and 1% GlutaMAX (Invitrogen; #35050) (hereafter referred to as Ad-DF+++). Biopsies were stored for a maximum of 24 hours at 4°C before being dissociated with sharp needles and digested with 10 μg/ml hyaluronidase (Sigma, #H4272) and 1.5 mg/mL collagenase (Sigma, #C6885). Cells were washed with Ad-DF+++ and cultured as described previously in CRC medium (with the exception of SB202190, now used at a final concentration of 10 μM in the medium) (Weeber, F. et al (2015) PNAS, Vol. 112, pages 13308-11).

PDOs were expanded to 2-3 million organoids and frozen down in a master biobank. PDO cultures were controlled for mycoplasma contamination every month using the MycoAlert Mycoplasma Detection Kit (Lonza). As part of quality control, PDOs were authenticated using a Taqman-based SNParray targeting 26 SNPs (Hartwig Medical Foundation). Identity scores of tumour DNA versus DNA obtained from healthy blood were computed as described elsewhere (Liang-Chu, M. M. Y. et a (2015), PLoS One 10, e0116218). PDOs with identity scores < 0.9 were discarded.

DNA-sequencing

Part of the biopsied material of each patient was used for routine clinical sequencing of a panel of cancer genes (Illumina TruSeq; ABL1; AKT1; ALK; APC; ATM; BRAF; CDH1; CDKN2A; CSF1R; CTNNB1; EGFR; ERBB2; ERBB4; FBXW7; FGFR1; FGFR2; FGFR3; FLT3; GNA11; GNAQ; GNAS; HNF1A; HRAS; ADH1; JAK2; JAK3; KDR; KIT; KRAS; MET; MLH1; MPL; NOTCH1; NPM1; NRAS; PDGFRA; PIK3CA; PTEN; PTPN11; RB1; RET; SMAD; SMARCB1; SMO; SRC; STK11; TP53; VHL) or whole-genome sequencing (WGS) by the Hartwig Medical Foundation (HMF). Both libraries were prepared according to manufactures instructions (targeted sequencing: FC-130-1008; WGS: Truseq Nano LT; FC-121-4001-3) and sequenced on the Illumina MiSeq (panel) or HiSeqX paired-end 2x150bp (WGS) platform. Analysis of the targeted panel was performed with Somatic Variant Caller v1.3 (Illumina). Analysis of the WGS data by the HMF was performed using their custom pipeline, which can be found here: https://github.com/hartwigmedical/pipeline/releases.

Drug screening

All drug screens were performed 2 times, once each by 2 independent researchers. PDOs (passage typically <10) were mechanically and enzymatically dissociated, filtered, and re-plated to allow for formation of organoids over the course of 4 days. After 4 days PDOs were collected, incubated with 1 mg/ml dispase II (Sigma #D4693) for 15 min to remove Geltrex, and counted using a hemocytometer and trypan blue. PDOs were resuspended in 1:3 Ad-DF+++:Geltrex at a concentration of 20 organoids/μl. Five μl/well of the suspension was dispensed in clear-bottomed, white-walled 96-well plates (Corning, #3707) using an automated repeat pipet and overlaid with 200 μl CRC medium.

We generated 6-step, 4-fold drug matrices of 5-FU + oxaliplatin or 5-FU + irinotecan and 10-step, 2-fold single

drug dose response curves in technical triplicate using a Tecan D300e digital dispenser (5-FU range = 0.319-200 μM; SN-38 range = 0.195-100 nM; oxaliplatin range = 0.391-200 μM). Read-out was performed at day 0 ('baseline') and at day 6 in the positive control (10 μM phenylarsine oxide), negative control (dimethyl sulfoxide at a concentration equal to the maximum present in drug-treated wells), and the drug-treated wells. Quantification of cell viability was done by replacing the CRC medium with 50 μL Cell-TiterGlo 3D (Promega, #G9681) mixed with 50 μL Ad-F+++ according to manufacturer instructions in an Infinite 200 Pro plate reader (Tecan Life Sciences).

Data analysis

For P1a-d, the results reported are the average of the 4 samples. In cases where lesion response could not be accurately measured (P1, P3.1, P14, and P21), overall response was used, as these two measures are highly correlated.

Drug-induced growth rate inhibition (GR) values were calculated based on median luminescence values of day 0, untreated day 6 and drug-treated wells at day 6, using the method described in more detail elsewhere (Hafner, M., et al (2016), Nat. Methods, Vol. 13, pages 521-527). For the FI (5-FU and irinotecan) cohort, the GR value of each data point in the whole drug combination matrix or 2 dose response curves (200 μM 5-FU anchor plus a titration of SN-38 combined with 6.25 nM SN-38 anchor and a titration of 5-FU) was summed to create a GR score for each PDO line. Z-scores used in the Kaplan-Meier curve were calculated as $(\mu - X)/\sigma$ ($\mu$ = mean; X = score; $\sigma$ = standard deviation). Scores for response to combined FO (5-FU and oxaliplatin) in vitro were calculated based on the 1:1 ratio (the 'diagonal' in the combination matrix) of FO, again summing the GR value of each point to create an overall score. Curve fitting and estimation of GR50s was done using a similar statistical setup as described previously, with the addition of the asymptote parameter (Hafner, M., et al (2016), Nat. Methods, Vol. 13, pages 521-527; Iorio, F. et al (2016), Cell, Vol. 166, pages 740-754).

The latter was allowed to co-vary on a drug level with the potency parameter xmid. The fits were performed using nlme in the statistical language R. AUCgs were inferred by integrating fitted curves. Correlation analyses, with associated p-values, were performed using a custom R-script. To analyse the reproducibility between drug screen 1 and drug screen 2, performed by 2 independent researchers, we calculated the Pearson's R and corresponding p-value using either the GR value of the 11 data points in the 2 complementary dose response curves identified as having the largest variance within our samples (FI set) or the GR value of the 36 data points in the full combination matrix (FO set).

To identify the 2 complementary dose response curves having the largest variance within our samples in the FI set, we calculated the variance for each of the 36 data points in the full drug matrix, across all 12 PDOs, in a leave-one-out setting. To set the empirically determined threshold for the FI classifier, the analyses were performed in a cross-validation setting. That is, the classified (test) cases were not used for classifier construction. For each sample, the GR scores (obtained by summing the GR values of each of the 11 data points in the 2 complementary dose response curves having the largest variance within our samples) of all other samples were used to determine the test threshold used to classify the left-out sample, based on lesion response.

To determine whether our empirically defined threshold performed better than chance, we randomly re-assigned our 12 GR scores among 6 sensitive and 6 resistant 'patients' 10,000 times. At each iteration, we assigned a new threshold as described above and assessed how well this threshold classified the randomized data. The p-value for our empirically determined threshold was calculated by dividing the number of cases where a randomly generated classifier threshold performed as well as, or better than, our empirically determined classifier threshold by the number of iterations executed.

To set the threshold used in our data set, we selected the highest GR score that correctly classified all sensitive patients, while correctly classifying the maximum number of resistant patients (GR>0.46). ROC curve plots, auROC curves, and confidence intervals of the auROC curves were generated using the PPROC v1.3.1 package in R. All other statistical tests were performed two-tailed in GraphPad Prism V7.03 or R.

Results

We embarked on a translational clinical study, the TUMOROID trial, to determine the feasibility and potential value of PDOs as a test for chemotherapy of CRC patients. The TUMOROID trial is a multi-centre, observational, prospective clinical trial, focusing on 2 chemotherapy combinations commonly used in colorectal cancer (CRC). Included patients could receive either infusional 5-FU or capecitabine (oral pro-drug of 5-FU), in combination with either oxaliplatin (referred to as FO) or irinotecan (referred to as FI). The primary endpoint was the development of a clinical test to accurately predict responsiveness to chemotherapy or said otherwise, identify non-responders and responders to chemotherapy (Fig. 1A). Non-responders were defined as patients with progressive disease after 3 cycles of chemotherapy according to RECIST 1.1 (Eisenhauer, E. A. et al (2009), Eur. J. Cancer, Vol. 45, pages 228-247).

We included 54 core needle biopsies from 48 patients and generated PDOs from these samples as previously

described (Weeber, F. et al (2015) PNAS, Vol. 112, pages 13308-11). Four patients did not undergo a biopsy and 2 patients were non-evaluable due to treatment-related toxicity. Our effort resulted in 29 PDO cultures from 23 patients. 19 cultures failed due to: too few tumour cells in the biopsy (n=6), quality control (n=5), no outgrowth (n=5), or bacterial infection (n=3).

Overall, we obtained about 60% PDO culture success rate, which is in line with previous reports (Fig. 1A) (Weeber, F. et al (2015) PNAS, Vol. 112, pages 13308-11; Vlachogiannis, G. et al (2018), Science, Vol. 359, pages 920-926). Culture success rate and time lines are likely to be further improved by obtaining multiple core biopsies, together with direct evaluation of the biopsies by a pathologist in order to identify samples with low cellularity (Vlachogiannis, G. et al (2018), Science, Vol. 359, pages 920-926).

Tumour characteristics, clinical background, pathological parameters, and genetic aberrations identified via sequencing are presented in Table 1 below. Of the 29 cultures, 13 were obtained prior to FO, 4 post FO, 3 post-FO/pre-FI, and 9 prior to FI treatment (Fig. 1A) and cultures were generated from the lesion locations indicated in Fig. 1B. The median time to establish a PDO master biobank of 2-3 million organoids was 38.5 days (quartile range about 23.4-70.3 days) and most cultures could be frozen down within 2-4 passages (Fig. 1C). Our initial assay used about 32,000 PDOs, allowing the majority of cultures to be subjected to large-scale drug screens within 2 months post-biopsy.

| TUMOR CHARACTERISTICS | | | | | | PREVIOUS TREATMENTS | |
|---|---|---|---|---|---|---|---|
| Study numb | Localization | Differentiation | Genetic profile<br>Mutations | MSI status | Biopsied lesion | Systemic<br>Treatment regimen | Local<br>Radiotherapy |
| P1 | Rectum | Moderately | ^ $BRAF^{V600E}$ | MSS | Skin | CAPOX<br>Irinotecan/bevacizumab<br>Trametinib/panitumumab | 5x5Gy (neoadjuvant)<br>Palliative RT on five lesions |
| P2.1 | Rectosigmoid | NA | - | MSS | Liver | CAPOX-B<br>Bevacizumab<br>Capecitabine | - |
| P2.2 | Rectosigmoid | NA | - | MSS | Liver | CAPOX-B<br>Bevacizumab<br>Capecitabine<br>FOLFOX | - |
| P3.1 | Colorectal NOS | Moderately | ^ $APC^{T1493R}$; $PIK3CA^{Q545L}$; $KRAS^{G12C}$ | MSS | Liver | - | - |
| P3.2 | Colorectal NOS | Moderately | ^ $APC^{T1493R}$; $KRAS^{G12C}$; $SMAD4^{R361C}$ | MSS | Liver | CAPOX-B | - |
| P4.1 | Sigmoid | NA | ^ $MEK1^{K57N}$ | MSS | Liver | - | - |
| P4.2 | Sigmoid | NA | ^ $MEK1^{K57N}$ | MSS | Liver | CAPOX-B | |
| P5 | Transverse colon | Poorly | * $PIK3CA^{E1100del}$; $TP53^{R209fs}$ | MSS | Liver | | |
| P6 | Sigmoid | Moderately | * $KRAS^{G12C}$; $SMAD4^{Q245*}$; $TP53^{R205D}$ | MSS | Liver | CAPOX-B | |
| P7 | Sigmoid | NA | * $TP53^{R209fs}$ | MSS | Liver | CAPOX-B | - |
| P8 | Rectum | NA | * $APC^{P2216T/G1367*}$; $KRAS^{A146T}$; $TP53^{R175H}$; $BARD1^{P182L}$; $CSF1R^{A150C}$; $MET^{A425H}$; $PTCH1^{A497T}$ | MSS | Lung | CAPOX-B<br>CAP-B<br>CAPOX | 5x5Gy (primary tumor) |
| P9 | Rectum/colon | NA | * $BRAF^{V600E}$ | MSI | Peritoneum | CAPOX | 3x5Gy (rectum) |
| P10 | Cecum | Moderately | * $KRAS^{G12A}$ | MSS | Liver | - | - |
| P11 | Cecum | NA | * $KRAS^{A146T}$; $AKT1^{E17K}$ | MSS | Liver | - | - |
| P12 | Cecum | Undifferentiated | * $KRAS^{G13D}$; $TP53^{V173L}$; $EGFR^{G724V}$ | MSS | Lymph node | CAPOX<br>Capecitabine | Rectum (curative, cumulative 50Gy) |
| P13 | Colorectal NOS | Moderately | NA | NA | Liver | - | - |
| P14 | Colorectal NOS | Moderately | NA | NA | NA | Capecitabine | - |
| P15 | Rectum | Moderately | * $NRAS^{G12C}$ | MSS | Liver | CAPOX-B<br>CAP-B<br>Capecitabine | Liver (curative, cumulative 25Gy) |
| P16 | Sigmoid | Poorly | * $MEK1^{Q56P}$ | NA | Lymph node | CAPOX<br>Oxaliplatin<br>Bevacizumab | - |
| P17 | Colon transversum | NA | * $APC^{R1450*}$; $BRAF^{V600E}$; $PIK3CA^{E545K}$ | MSS | Liver | CAPOX-B<br>CAP-B<br>Bevacizumab | - |
| P18 | Rectum | NA | - | MSS | Lymph node | CAPOX-B<br>Bevacizumab<br>Capecitabine<br>Panitumumab | Rectum (adjuvant, cumulative 50Gy) |
| P19 | Rectum | Poorly | $APC^{G1288X}$; $KRAS^{G13A}$; $BRAF^{D594G}$; $TP53^{Q266K}$ | NA | Breast | Capecitabine<br>CAPOX-B<br>CAP-B | Rectum (curative, cumulative 50Gy)<br>Regional lymph nodes (curative, cumulative 50Gy) |
| P20 | Rectum | Moderately | $APC^{R564*/P1439fs*34}$; $KRAS^{G12A}$; $TP53^{C141Y}$; $ERBB3^{E332K}$; $FBXW7^{R465C}$ | MSS | Liver | Capecitabine (neoadjuvant)<br>CAPOX-B | Rectum (neoadjuvant, cumulative 45-50Gy) |
| P21 | Rectum | Moderately | $TP53^{G294*}$; $PIK3CA^{A38H}$; $EGFR^{A521L}$; $SMAD4^{A537H}$ | MSS | Primary | Capecitabine<br>CAPOX-B | Rectum (neoadjuvant, cumulative 50.4Gy) |
| P22 | Sigmoid | Moderately | - | NA | Omentum | FOLFOX<br>CAPOX-B<br>CAP-B<br>Panitumumab | Osseous metastasis (palliative, cumulative 8Gy) |
| P23 | Rectosigmoid | Moderately | $APC^{E1306/E1353*}$; $ARAF^{D46N}$; $MSH2^{L93}$; $FBXW7^{G557V}$; $PALB2^{V425M}$ | MSS | Liver | FOLFOX<br>Bevacizumab | Osseous metastasis T4 (palliative, cumulative 30Gy) |

**Table 1.** Genetic, pathological, and clinical characteristics of cohort of patients with metastatic colorectal cancer.

We first tested PDOs from 12 patients treated with FI (Fig. 2A). After 3 cycles, 6 patients had progressive disease (PD), 3 had stable disease (SD), and 2 had partial remissions (PR) (Fig. 2B). One patient had a mixed response:

the biopsied lesion was stable, but the patient was clinically progressive due to the appearance of new lesions. This distribution of responses is in line with larger studies using FI (Guglielmi, A. P. and Sobrero, A. F. (2007), Cancer Res., Vo. 1, Pages 57-63). Because FO and FI are combination chemotherapies of 2 drugs, which each undergo clearance in patients at different rates, are metabolized (e.g. irinotecan to SN38), and have peak concentrations (Cmax) rather than steady state concentrations, we designed an extensive matrix of pharmacokinetically relevant drug concentrations covering both combination and single-agent treatments.

Following this design, we exposed PDOs to either FI or FO chemotherapy for 6 days and calculated the drug-induced growth rate inhibition (GR) of each condition 6 days post-drug exposure (Hafner, M., et al (2016), Nat. Methods, Vol. 13, pages 521-527; Harris, L. A. et al (2016), Nat. Methods, Vol. 13, pages 497-500) (Fig. 2C). Each screen was repeated by a second person to control for inter-observer bias. Biological replicates were highly concordant between researchers (average Pearson's R = 0.96; range 0.90-0.98; Fig. 2D), demonstrating that the PDO drug screen pipeline generated reproducible data.

We quantified responses to the combination chemotherapy by summing the GR score across the full matrix of concentrations of FI and observed a non-significant trend of patients with PD being more resistant to these drugs in vitro (p=0.0649; Fig. 2E). Then, we refined the drug assay by determining the concentrations of FI at which the variance ('window') of in vitro chemosensitivity was the largest. This analysis identified 2 complementary dose response curves - 200 $\mu$M 5-FU as anchor and a titration of SN-38, combined with 6.25 nM SN-38 as anchor and a titration of 5-FU. Using this sub-matrix, in vitro chemosensitivity was significantly different in cultures derived from PD versus PR/SD lesions (p=0.0260; Fig. 2F and Fig. 2C).

We next determined the ability of PDOs to predict patient response. Receiver operating characteristic (ROC) curve generated from in vitro responses harboured area under the curves (auROCs) of 0.89 for lesion response (Fig. 2G) and 0.86 for overall response (Fig. 2G), suggesting PDOs have predictive value in this setting. Additionally, patients with the 50% most sensitive PDOs (z-score < 0) had a significantly higher progression free survival (PFS), indicating that in vitro sensitivity to FI is associated with a longer response (Fig. 2H). Finally, the sub-matrix performed better than other in vitro endpoints (summarized in Fig. 2I).

We next aimed to construct a classifier to accurately identify non-responders, without misclassifying responders. To prevent overfitting, we performed these analyses in a cross-validation setting, so that the classified (test) cases were not used for classifier construction. Briefly, for each sample, the GR scores (derived from the sub-matrix identified above) of all other samples were used to determine the test threshold used to classify the left-out sample. Using this threshold, we correctly classified 100% of (left-out) sensitive patients and 83% of resistant patients (empirical p-value = 0.0017). To set the threshold used in our data set, we selected the highest GR score that correctly classified all sensitive patients, while correctly classifying the maximum number of resistant patients (GR > 0.46).

These data demonstrate that we identified optimal settings to distinguish non-responders in vitro and that PDO tests for combination chemotherapy should consider the interaction between drugs, rather than commonly used in vitro parameters of single drug activity (Fig. 2I) (Tiriac, H. et al (2018) Cancer Discov., CD-18-0349 doi:10.1158/2159-8290.CD-18-0349). Importantly for clinical implementation, the newly 'designed' dose response curves only require 9,200 PDOs, which can be readily generated within 4 weeks.

We then performed similar experiments to test the predictive value of PDOs for FO chemotherapy using 20 PDOs derived from 14 patients (Fig. 3A). Thirteen biopsies were taken before start of treatment and 7 after clinical progression on FO, 4 of which were generated from patients with a mixed response to FO (Fig. 3B) (Eisenhauer, E. A. et al (2009), Eur. J. Cancer, Vol. 45, pages 228-247). All samples were screened and analysed as previously described for FI and replicates were highly reproducible (Fig 3C).

In contrast to the FI set, we did not find a significant difference in PDOs generated from PD versus PR/SD lesions in response to any of the tested parameters (Fig. 3D and Fig. 3E). Consequently, the ROC curve showed no predictive value (Fig. 3F-G).

To date, there are no predictive tests for (non-)responsiveness to standard-of-care chemotherapy in colorectal cancer (CRC). Although several biomarkers have been proposed for 5-FU- and irinotecan-based regimens, these are currently not used in clinical practice (Bosch, L. J. W. et al (2016), Clin. Cancer Res., Vol. 22, pages 4612-22; Aschele, C. et al (1999), J. Clin. Oncol., Vol. 17, pages 1760-1760; Ebert, M. P. A. et al (2012), N. Engl. J. Med., Vol. 366, pages 44-53; Haan, J. C. et al (2014), Nat. Commun., Vol. 5, page 5457). Here we provide evidence that PDO response to FI in vitro is correlated to patient response in vivo (Fig. 4).

Our data also support the use of the method as taught herein as a predictive test for the failure (for identifying non-responders to FI) of FI palliative chemotherapy in patients with metastatic CRC as well as a predictive test for the success (for identifying responders to FI) of FI palliative chemotherapy in patients with metastatic CRC.

Having now fully described this invention, it will be appreciated by those skilled in the art that the same can be performed within a wide range of equivalent parameters, concentrations, and conditions without departing from the spirit and scope of the invention and without undue experimentation.

All references cited herein, including journal articles or abstracts, published or corresponding patent applications,

patents, or any other references, are entirely incorporated by reference herein, including all data, tables, figures, and text presented in the cited references. Additionally, the entire contents of the references cited within the references cited herein are also entirely incorporated by references.

Reference to known method steps, conventional methods steps, known methods or conventional methods is not in any way an admission that any aspect, description or embodiment of the present invention is disclosed, taught or suggested in the relevant art.

The foregoing description of the specific embodiments will so fully reveal the general nature of the invention that others can, by applying knowledge within the skill of the art (including the contents of the references cited herein), readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein.

It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

## Claims

**1.** A (in vitro) method for determining a reference value 1 (REF1) to segregate a first group of patients and a second group of patients, wherein the first group and second group of patients are composed of gastrointestinal cancer patients who are responders and non-responders, respectively, to a therapeutic treatment comprising 5-fluorouracil or capecitabine in combination with irinotecan or irinotecan alone, said method comprising the step of:

a. Providing a tumor sample obtained from each gastrointestinal cancer patient in the first and second group of patients;
b. Generating a plurality of patient-derived tumor organoids (PDOs) from the tumor sample obtained from each gastrointestinal cancer patient in the first and second group of patients, thereby obtaining a PDO line for each gastrointestinal cancer patient in the first and second group of patients;
c. Contacting the PDO line of each gastrointestinal cancer patient in the first and second group of patients with 5-fluorouracil or capecitabine in combination with irinotecan or irinotecan alone at a first dosage, and optionally at a second or further dosage(s), for at least 3 days, preferably 6 days;
d. Calculating a drug-induced growth rate inhibition (GR) value for the PDO line of each gastrointestinal cancer patient in the first and second group of patients for the first dosage, and optionally for the second dosage or further dosage(s);
e. Calculating a GR score for the PDO line of each gastrointestinal cancer patient in the first and second group of patients, wherein the GR score is the GR value for the PDO line of each gastrointestinal cancer patient in the first and second group of patients for the first dosage or wherein the GR score is calculated by summing the GR value of the PDO line of each gastrointestinal cancer patient in the first and second group of patients for the first dosage and second dosage or further dosages, thereby obtaining a GR score for each gastrointestinal cancer patient in the first and second group of patients;
f. Establishing the REF1 value, wherein said REF1 is the highest GR score in the first group of patients that segregates a maximum number of gastrointestinal cancer patients in the first group of patients from a maximum of the gastrointestinal cancer patients in the second group of patients;

or
wherein said REF1 is the lowest GR score in the second group of patients that segregates a maximum number of gastrointestinal cancer patients in the second group of patients from a maximum of the gastrointestinal cancer patients in the first group of patients;
or
wherein said REF1 is a GR score that is between the highest GR score in the first group of patients and the lowest GR score in the second group of patients, wherein said GR score segregates a maximum number of gastrointestinal cancer patients in the first group of patients from a maximum of the gastrointestinal cancer patients in the second group of patients.

**2.** An (in vitro) method for predicting responsiveness or treatment outcome of a gastrointestinal cancer patient to a chemotherapeutic treatment comprising 5-fluorouracil or capecitabine in combination with irinotecan or irinotecan alone, said method comprising the steps of:

a. Providing a plurality of patient-derived tumor organoids (PDOs) obtained from said gastrointestinal cancer patient, thereby obtaining a PDO line for said gastrointestinal cancer patient;
b. Contacting the PDO line of the gastrointestinal cancer patient with 5-fluorouracil or capecitabine in combination with irinotecan or irinotecan alone at a first dosage and optionally at second dosage or further dosage (s) for at least 3 days;
c. Calculating a drug-induced growth rate inhibition (GR) value for the PDO line of said gastrointestinal cancer patient for the first dosage, and optionally for the second dosage or further dosage(s);
d. Calculating a GR score for the PDO line of said gastrointestinal cancer patient, wherein the GR score is the GR value for the PDO line of said patient for the first dosage or wherein the GR score is calculated by summing the GR value of the PDO line of said gastrointestinal cancer patient for the first dosage and second dosage or further dosages, thereby obtaining a GR score for said gastrointestinal cancer patient; and
e. Predicting responsiveness or treatment outcome of the gastrointestinal cancer patient to the chemotherapeutic treatment comprising 5-fluorouracil or capecitabine in combination with irinotecan or irinotecan alone, wherein: i) if the GR score of the PDO line for said gastrointestinal cancer patient is equal or greater than a reference 1 (REF1) value, than it indicates that said gastrointestinal cancer patient is a non-responder or is less likely to respond to the chemotherapeutic treatment comprising 5-fluorouracil or capecitabine in combination with irinotecan or irinotecan alone; or ii) if the GR score of the PDO line for said gastrointestinal cancer patient is lower than a REF1 value, than it indicates that said gastrointestinal cancer patient is a responder or is more likely to respond to the chemotherapeutic treatment comprising 5-fluorouracil or capecitabine in combination with irinotecan or irinotecan alone; wherein the REF 1 value is determined according to the method of claim 1;
f. optionally, repeating steps (a) to (e) for subsequent PDO lines from said gastrointestinal cancer patient

**3.** Method according to claim 1 or 2, wherein the gastrointestinal cancer patient suffers from a gastrointestinal cancer selected from esophagus cancer, stomach cancer, biliary system cancer, small intestine cancer, large intestine cancer (or colon cancer), pancreatic cancer, colorectal cancer, rectum cancer and anus cancer.

**4.** Method according to claim 3, wherein the gastrointestinal cancer is a metastatic gastrointestinal cancer.

**5.** Method to claim 3 or 4, wherein the gastrointestinal cancer is colorectal cancer or pancreatic cancer.

**6.** Method according to any of the previous claims, wherein the gastrointestinal cancer patient is naive to the chemotherapy treatment comprising 5-fluorouracil or capecitabine in combination with irinotecan or irinotecan alone.

**7.** Method according to any of the previous claims, wherein the PDO line for each gastrointestinal cancer patient is contacted with 5-fluorouracil or capecitabine in combination with irinotecan or irinotecan alone for a period of time ranging from 3 days to 15 days, or for 3, 4, 5, 6, 7, 8, 9, 10, 11, 12, 13, 14 or 15 days, preferably 6 days.

**8.** Method according to any of the previous claims, wherein the PDO line for each gastrointestinal cancer patient is derived from a primary tumor or a metastatic lesion within each gastrointestinal cancer patient.

**9.** Method according to claim 8, wherein the metastatic lesion is taken from a region in the body of each gastrointestinal cancer patient that is selected from skin, lung, lymph node, liver, breast, peritoneum, and omentum.

**10.** Method according to any of the previous claims, wherein the chemotherapeutic treatment further comprises one or more therapeutic compounds, and wherein contacting the PDO line for each gastrointestinal cancer patient further comprises one or more therapeutic compounds.

**11.** Method according to claim 10 wherein the one or more therapeutic compounds is folinic acid and/or bevacizumab.

**12.** Method according to any one of the previous claims, wherein the PDO line of each gastrointestinal cancer patient is contacted with 5-fluorouracil or capecitabine in combination with irinotecan, simultaneously or sequentially.

**13.** 5-fluorouracil or capecitabine in combination with irinotecan or irinotecan alone for use in a method for treating a gastrointestinal cancer patient, said method comprising predicting responsiveness or treatment outcome of a gastrointestinal cancer patient to a chemotherapeutic treatment comprising 5-fluorouracil or capecitabine in combination with irinotecan or irinotecan alone, said method comprising the steps of:

a. Providing a plurality of patient-derived tumor organoids (PDOs) obtained from said gastrointestinal cancer

patient, thereby obtaining a PDO line for said gastrointestinal cancer patient;

b. Contacting the PDO line of the gastrointestinal cancer patient with 5-fluorouracil or capecitabine in combination with irinotecan or irinotecan alone at a first dosage and optionally at second dosage or further dosage (s) for at least 3 days;

c. Calculating a drug-induced growth rate inhibition (GR) value for the PDO line of said gastrointestinal cancer patient for the first dosage, and optionally for the second dosage or further dosage(s);

d. Calculating a GR score for the PDO line of said gastrointestinal cancer patient, wherein the GR score is the GR value for the PDO line of said patient for the first dosage or wherein the GR score is calculated by summing the GR value of the PDO line of said gastrointestinal cancer patient for the first dosage and second dosage or further dosages, thereby obtaining a GR score for said gastrointestinal cancer patient; and

e. Predicting responsiveness or treatment outcome of the gastrointestinal cancer patient to the chemotherapeutic treatment comprising 5-fluorouracil or capecitabine in combination with irinotecan or irinotecan alone, wherein: i) if the GR score of the PDO line for said gastrointestinal cancer patient is equal or greater than a reference 1 (REF1) value, than it indicates that said gastrointestinal cancer patient is a non-responder or is less likely to respond to the chemotherapeutic treatment comprising 5-fluorouracil or capecitabine in combination with irinotecan or irinotecan alone; or ii) if the GR score of the PDO line for said gastrointestinal cancer patient is lower than a REF1 value, than it indicates that said gastrointestinal cancer patient is a responder or is more likely to respond to the chemotherapeutic treatment comprising 5-fluorouracil or capecitabine in combination with irinotecan or irinotecan alone; wherein the REF 1 value is determined according to the method of claim 1.

f. optionally, treating the gastrointestinal cancer patient of step i with a treatment that does not comprise 5-fluorouracil or capecitabine in combination with irinotecan or irinotecan alone;

g. optionally, treating the gastrointestinal patient of step ii with a chemotherapeutic treatment comprising 5-fluorouracil or capecitabine in combination with irinotecan or irinotecan alone;

h. optionally, repeating steps (a) to (g) for subsequent PDO lines from said gastrointestinal cancer patient.

**14.** Method of treating a gastrointestinal cancer patient, said method comprising predicting responsiveness or treatment outcome of a gastrointestinal cancer patient to a chemotherapeutic treatment comprising 5-fluorouracil or capecitabine in combination with irinotecan or irinotecan alone, said method comprising the steps of:

a. Providing a plurality of patient-derived tumor organoids (PDOs) obtained from said gastrointestinal cancer patient, thereby obtaining a PDO line for said gastrointestinal cancer patient;

b. Contacting the PDO line of the gastrointestinal cancer patient with 5-fluorouracil or capecitabine in combination with irinotecan or irinotecan alone at a first dosage and optionally at second dosage or further dosage (s) for at least 3 days;

c. Calculating a drug-induced growth rate inhibition (GR) value for the PDO line of said gastrointestinal cancer patient for the first dosage, and optionally for the second dosage or further dosage(s);

d. Calculating a GR score for the PDO line of said gastrointestinal cancer patient, wherein the GR score is the GR value for the PDO line of said patient for the first dosage or wherein the GR score is calculated by summing the GR value of the PDO line of said gastrointestinal cancer patient for the first dosage and second dosage or further dosages, thereby obtaining a GR score for said gastrointestinal cancer patient; and

e. Predicting responsiveness or treatment outcome of the gastrointestinal cancer patient to the chemotherapeutic treatment comprising 5-fluorouracil or capecitabine in combination with irinotecan or irinotecan alone, wherein: i) if the GR score of the PDO line for said gastrointestinal cancer patient is equal or greater than a reference 1 (REF1) value, than it indicates that said gastrointestinal cancer patient is a non-responder or is less likely to respond to the chemotherapeutic treatment comprising 5-fluorouracil or capecitabine in combination with irinotecan or irinotecan alone; or ii) if the GR score of the PDO line for said gastrointestinal cancer patient is lower than a REF1 value, than it indicates that said gastrointestinal cancer patient is a responder or is more likely to respond to the chemotherapeutic treatment comprising 5-fluorouracil or capecitabine in combination with irinotecan or irinotecan alone; wherein the REF 1 value is determined according to the method of claim 1.

f. optionally, treating the gastrointestinal patient of step i with a treatment that does not comprise 5-fluorouracil or capecitabine in combination with irinotecan or irinotecan alone;

g. optionally, treating the gastrointestinal patient of step ii with a chemotherapeutic treatment comprising 5-fluorouracil or capecitabine in combination with irinotecan or irinotecan alone;

h. optionally, repeating steps (a) to (g) for subsequent PDO lines from said gastrointestinal cancer patient.

Fig. 1A

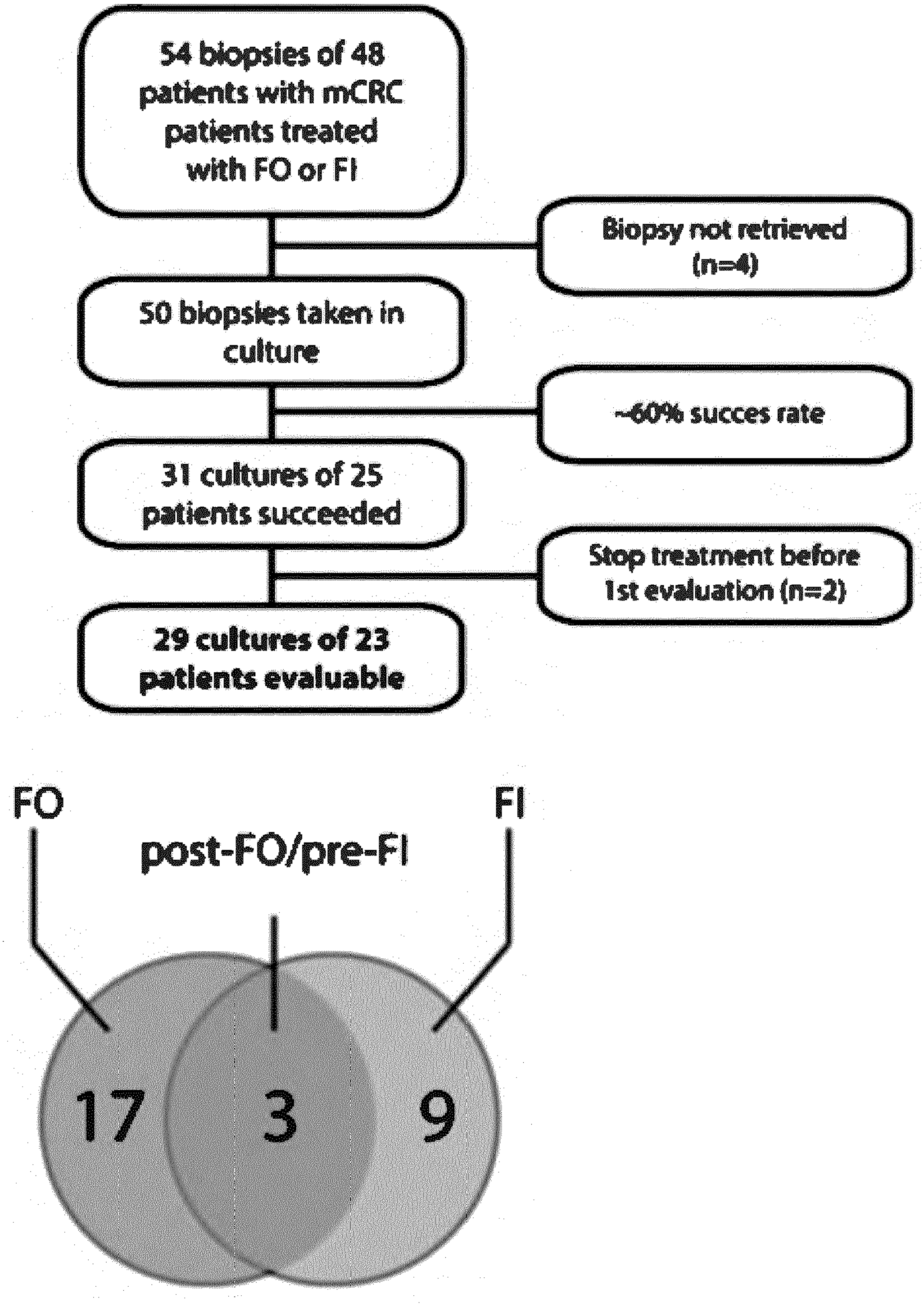
54 biopsies of 48 patients with mCRC patients treated with FO or FI
Biopsy not retrieved (n=4)
50 biopsies taken in culture
~60% succes rate
31 cultures of 25 patients succeeded
Stop treatment before 1st evaluation (n=2)
29 cultures of 23 patients evaluable
FO
FI
post-FO/pre-FI
17
3
9

Fig. 1B

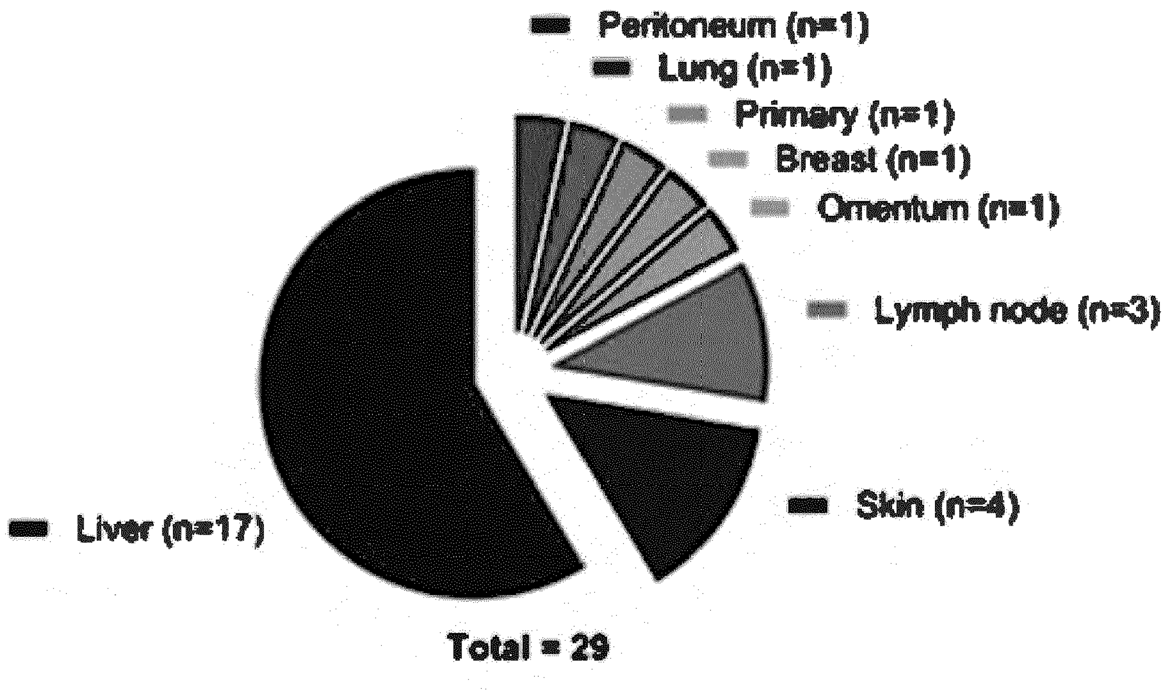
Peritoneum (n=1)
Lung (n=1)
Primary (n=1)
Breast (n=1)
Omentum (n=1)
Lymph node (n=3)
Skin (n=4)
Liver (n=17)
Total = 29

Fig. 1C

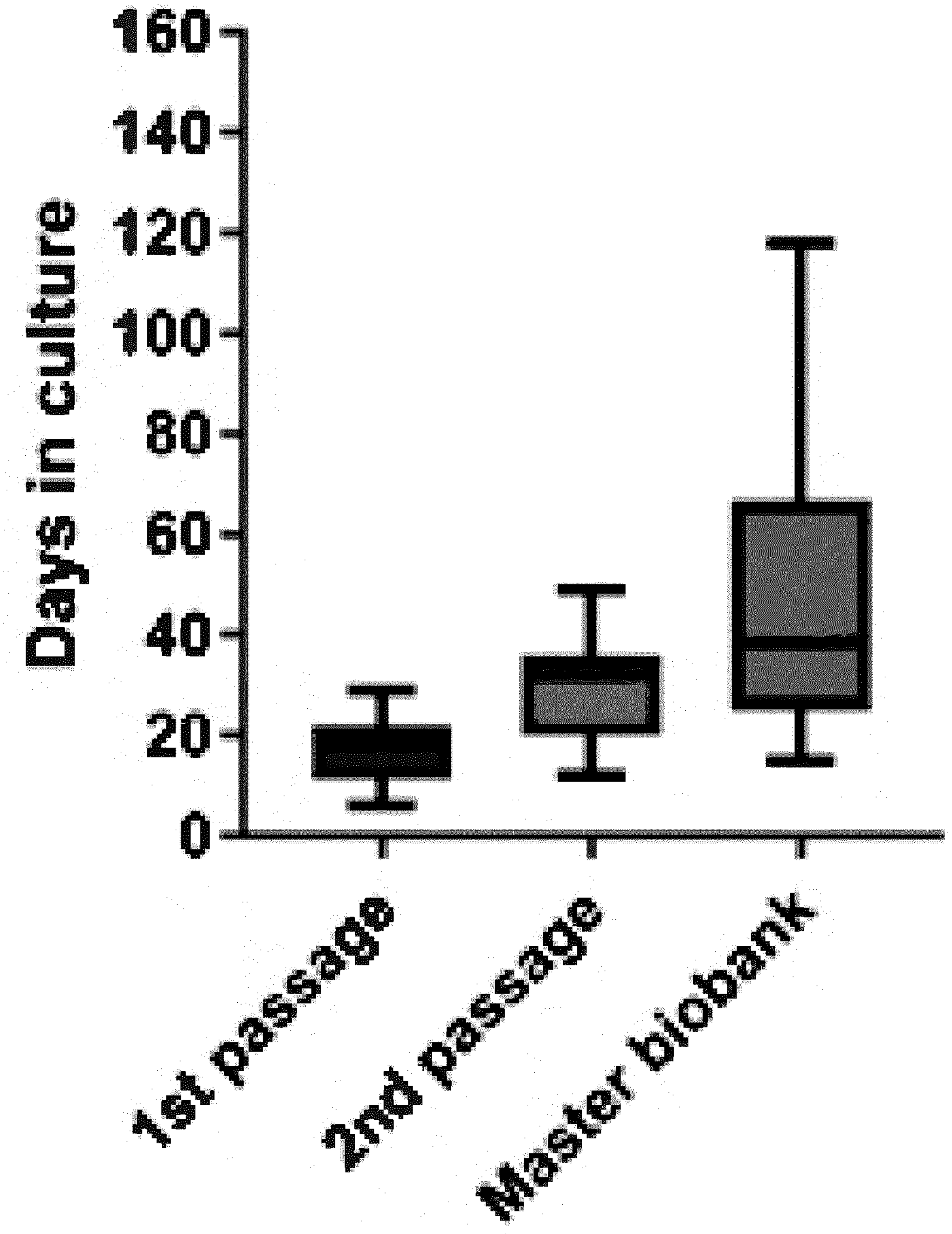
Days in culture
160
140
120
100
80
60
40
20
0
1st passage
2nd passage
Master biobank

Fig. 2A

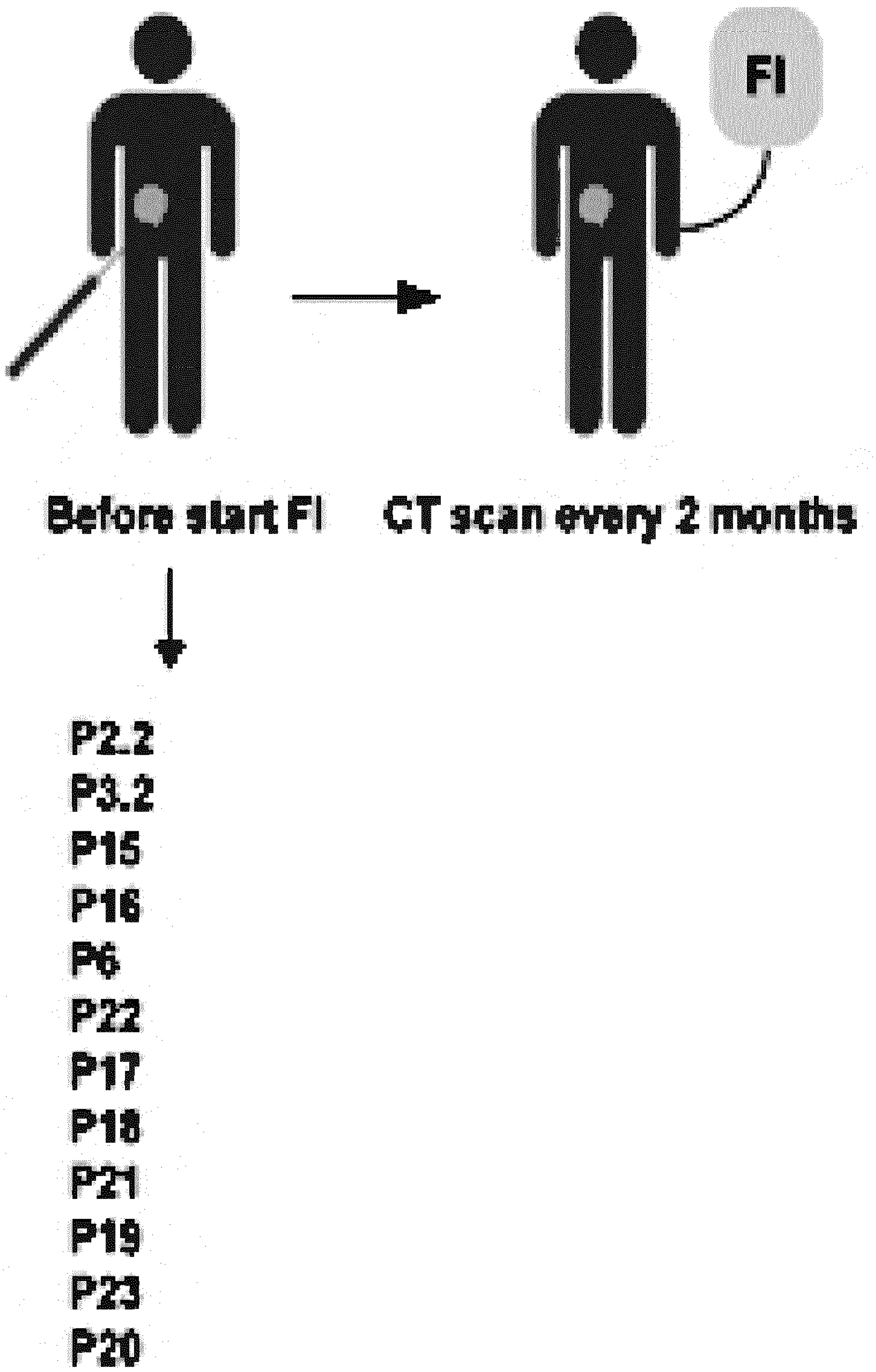
FI
Before start FI
CT scan every 2 months
P2.2
P3.2
P15
P16
P6
P22
P17
P18
P21
P19
P23
P20

Fig. 2B

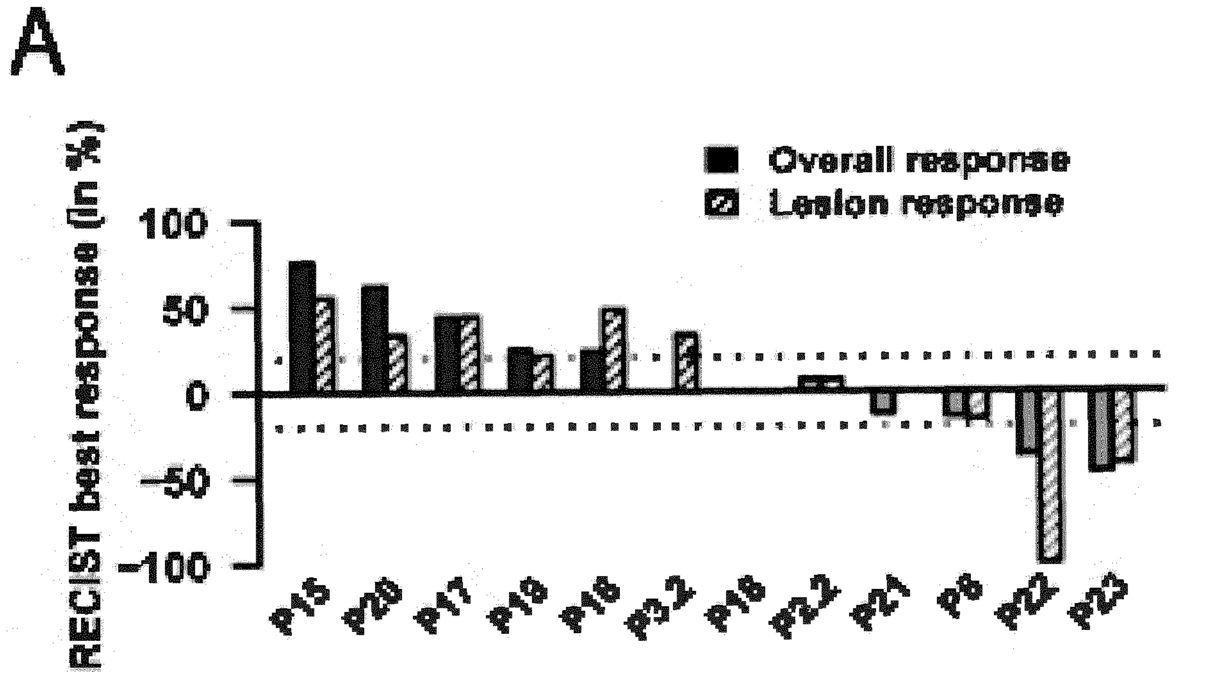
A
Overall response
Lesion response
RECIST best response (in %)
100
50
0
-50
-100
P15
P20
P17
P19
P16
P3.2
P18
P2.2
P21
P8
P22
P23

Fig. 2C

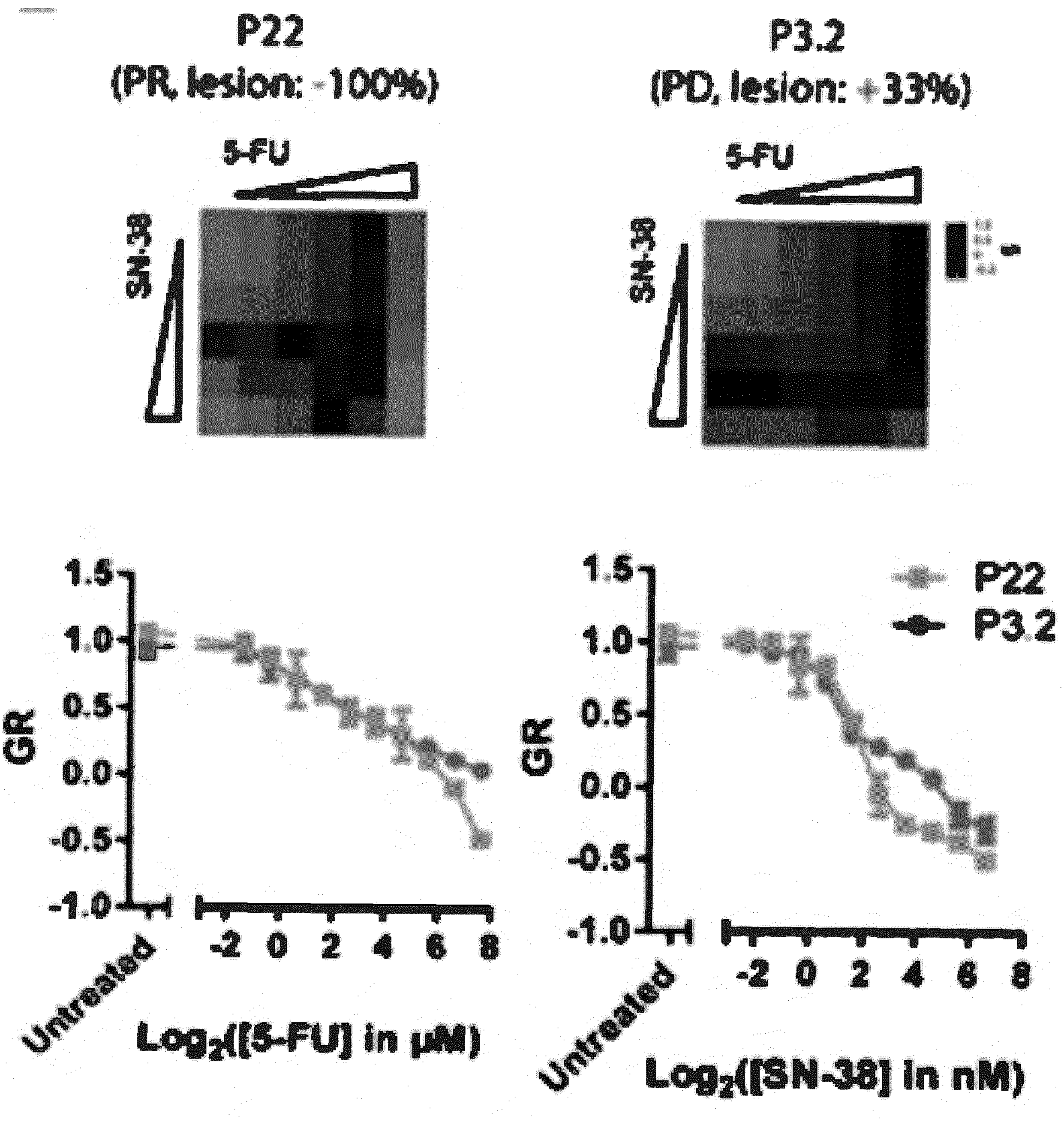
P22
(PR, lesion: -100%)
5-FU
SN-38
P3.2
(PD, lesion: +33%)
5-FU
SN-38
1.5
1.0
0.5
0.0
-0.5
-1.0
GR
Untreated
-2 0 2 4 6 8
Log2([5-FU] in μM)
P22
P3.2
Log2([SN-38] in nM)

Fig. 2D

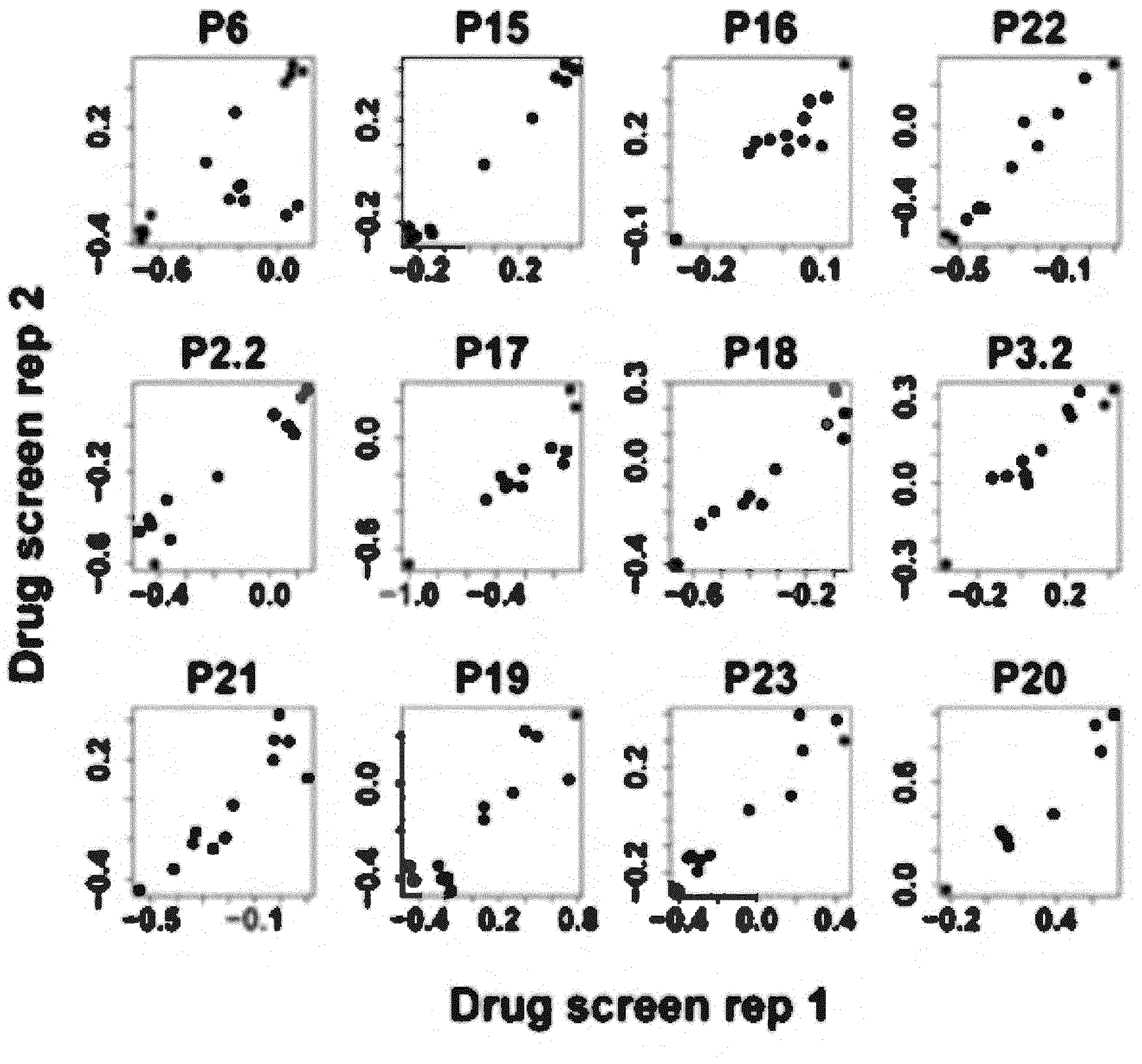
P6
P15
P16
P22
P2.2
P17
P18
P3.2
P21
P19
P23
P20
Drug screen rep 2
Drug screen rep 1

**Fig. 2E**

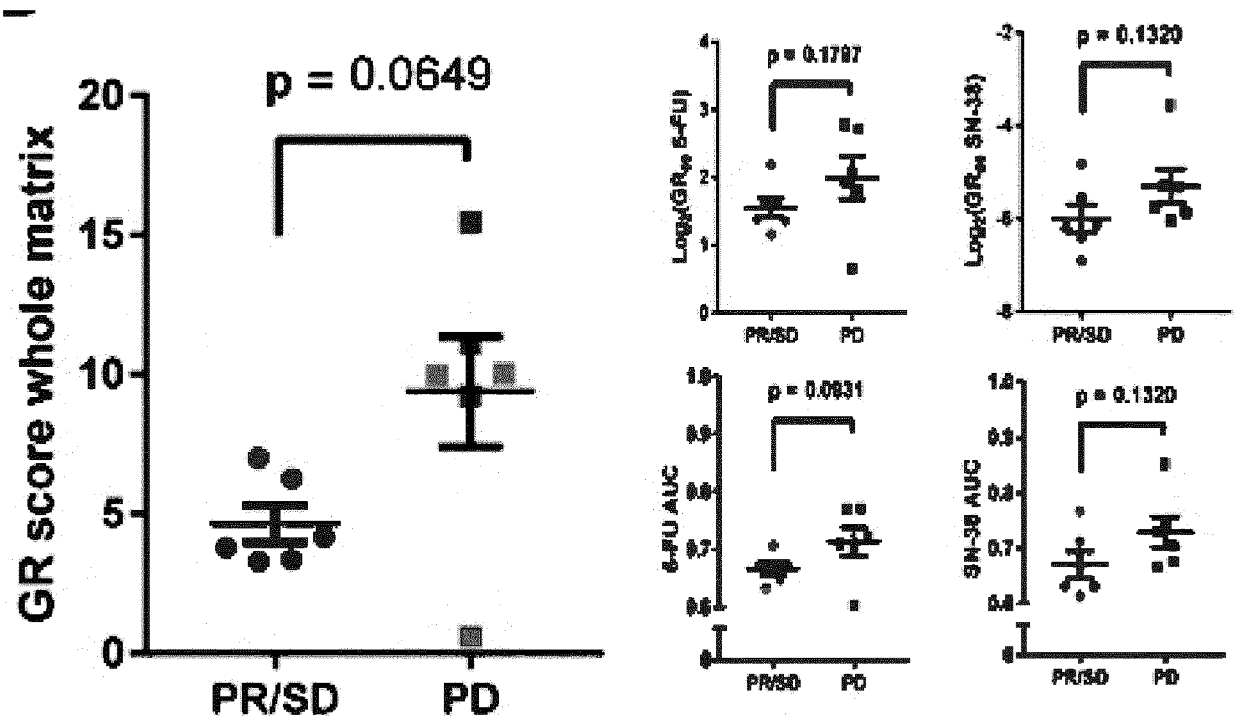

p = 0.0649
GR score whole matrix
20
15
10
5
0
PR/SD
PD
p = 0.1797
PR/SD
PD
p = 0.1320
PR/SD
PD
p = 0.0931
5-FU AUC
PR/SD
PD
p = 0.1320
SN-38 AUC
PR/SD
PD

Fig. 2F

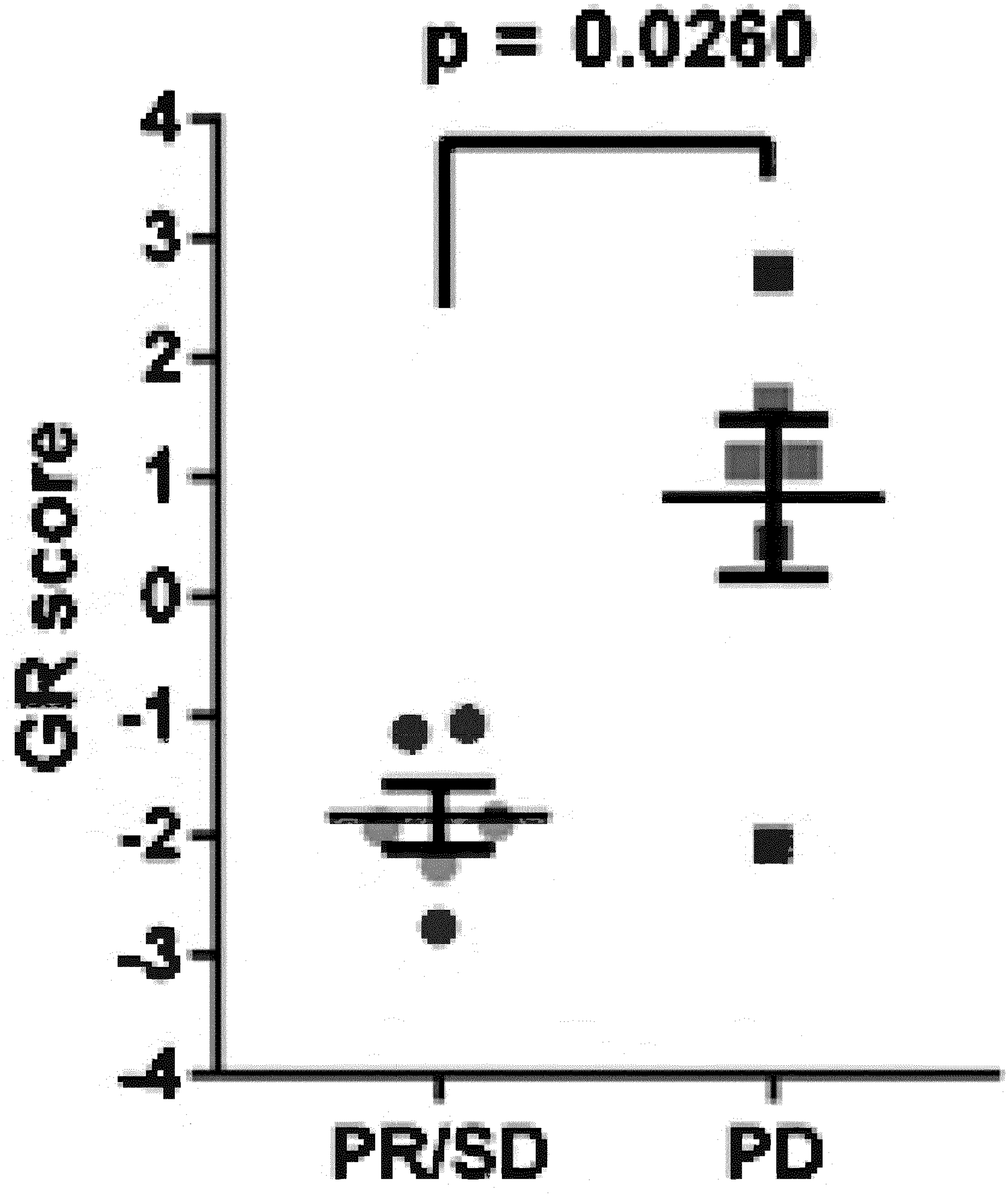

Fig. 2G

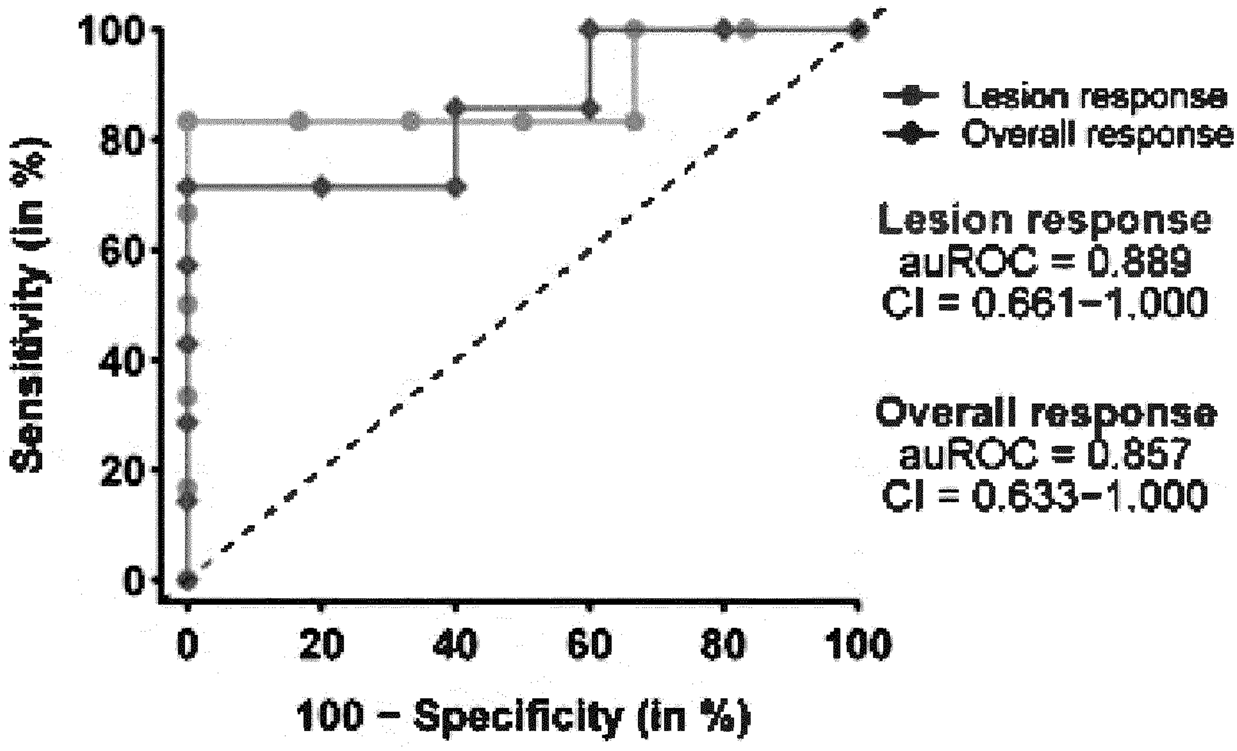
100
80
60
40
20
0
Sensitivity (in %)
0
20
40
60
80
100
100 − Specificity (in %)
Lesion response
Overall response
Lesion response
auROC = 0.889
CI = 0.661−1.000
Overall response
auROC = 0.857
CI = 0.633−1.000

Fig. 2H

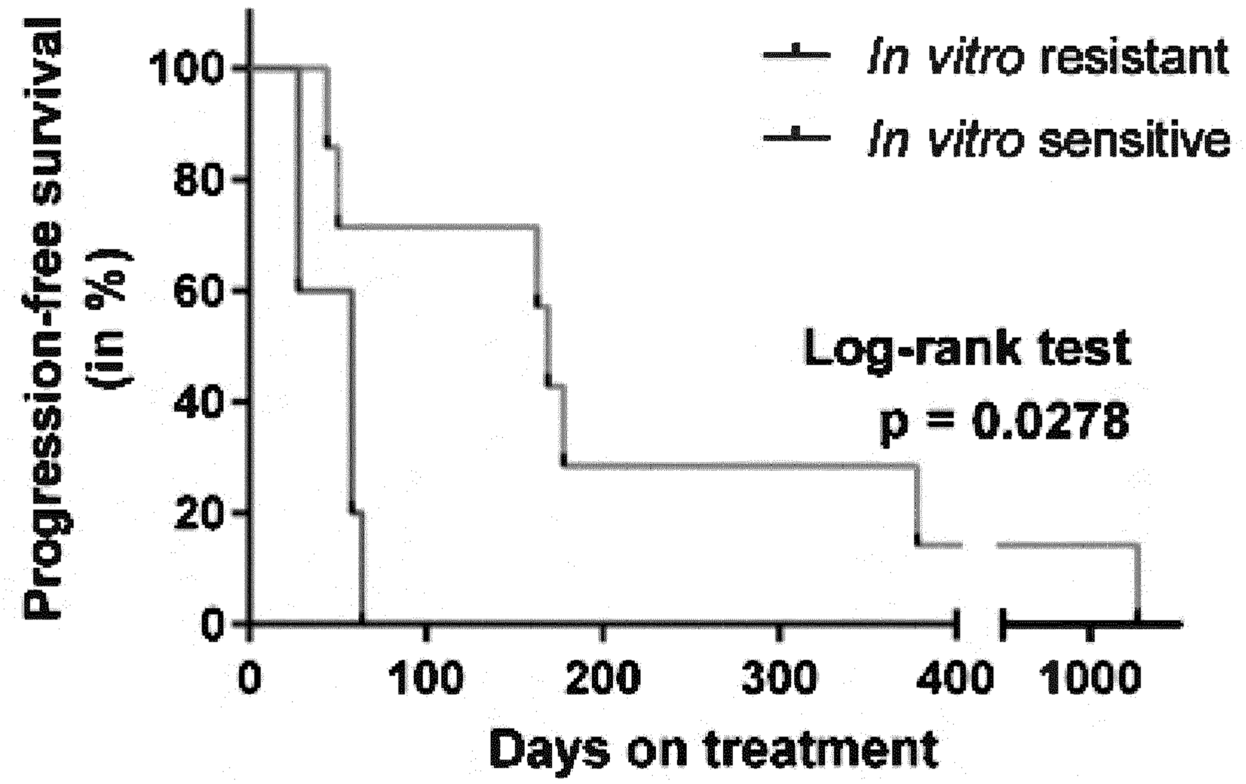

Progression-free survival (in %)
100
80
60
40
20
0
In vitro resistant
In vitro sensitive
Log-rank test
p = 0.0278
0
100
200
300
400
1000
Days on treatment

Fig. 2I

| Variable | Response | auROC | 95% CI |
|---|---|---|---|
| Sub-matrix | Lesion | 0.8889 | 0.6605 to 1.105 |
| Sub-matrix | Overall | 0.8571 | 0.6331 to 1.076 |
| Whole matrix | Lesion | 0.8333 | 0.5351 to 1.132 |
| Whole matrix | Overall | 0.7429 | 0.4315 to 1.054 |
| $GR_{50}$ 5-FU | Lesion | 0.6346 | 0.3434 to 0.9259 |
| $GR_{50}$ 5-FU | Overall | 0.6389 | 0.3483 to 0.9295 |
| $AUC_g$ 5-FU | Lesion | 0.8 | 0.4494 to 1.151 |
| $AUC_g$ 5-FU | Overall | 0.7 | 0.3484 to 1.052 |
| $GR_{50}$ SN-38 | Lesion | 0.6923 | 0.4388 to 0.9458 |
| $GR_{50}$ SN-38 | Overall | 0.6667 | 0.3937 to 0.9396 |
| $AUC_g$ SN-38 | Lesion | 0.7 | 0.3192 to 1.081 |
| $AUC_g$ SN-38 | Overall | 0.5333 | 0.1739 to 0.8928 |

Fig. 3A

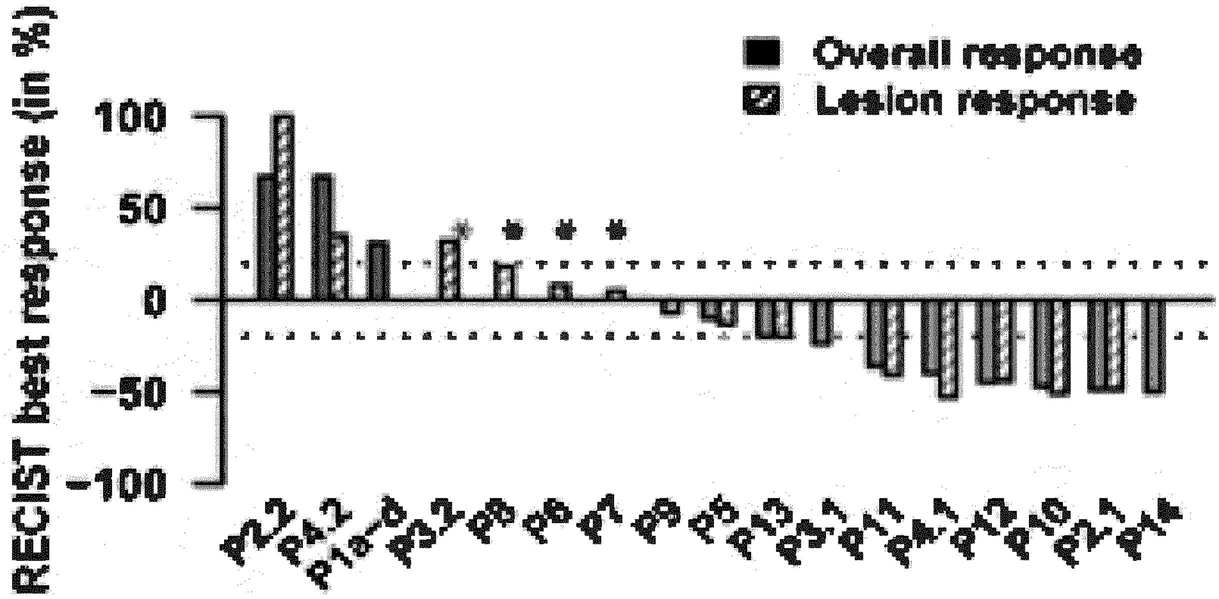
Overall response
Lesion response
RECIST best response (in %)
100
50
0
-50
-100
P2.2
P4.2
P1a-d
P3.2
P8
P6
P7
P9
P5
P13
P3.1
P11
P4.1
P12
P10
P2.1
P14

Fig. 3B

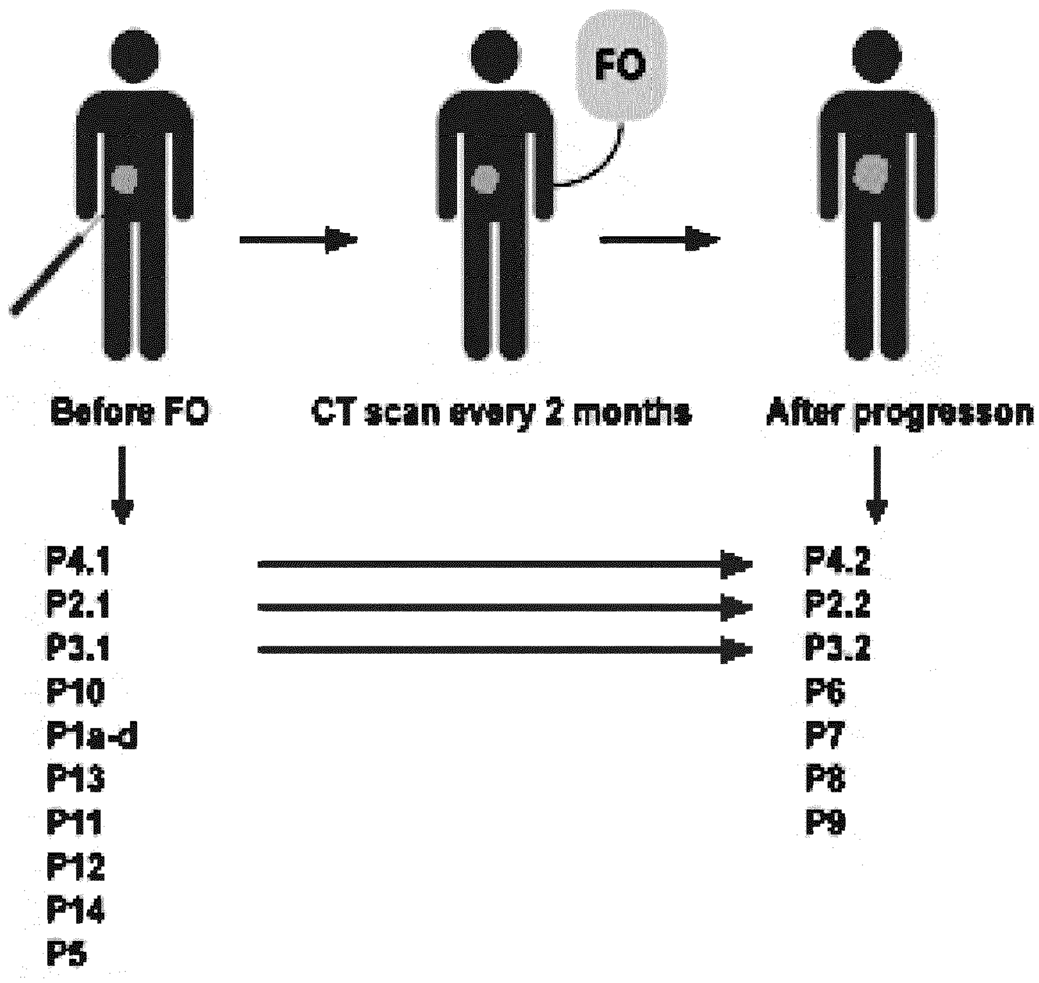
FO
Before FO
CT scan every 2 months
After progresson
P4.1
P2.1
P3.1
P10
P1a-d
P13
P11
P12
P14
P5
P4.2
P2.2
P3.2
P6
P7
P8
P9

Fig. 3C

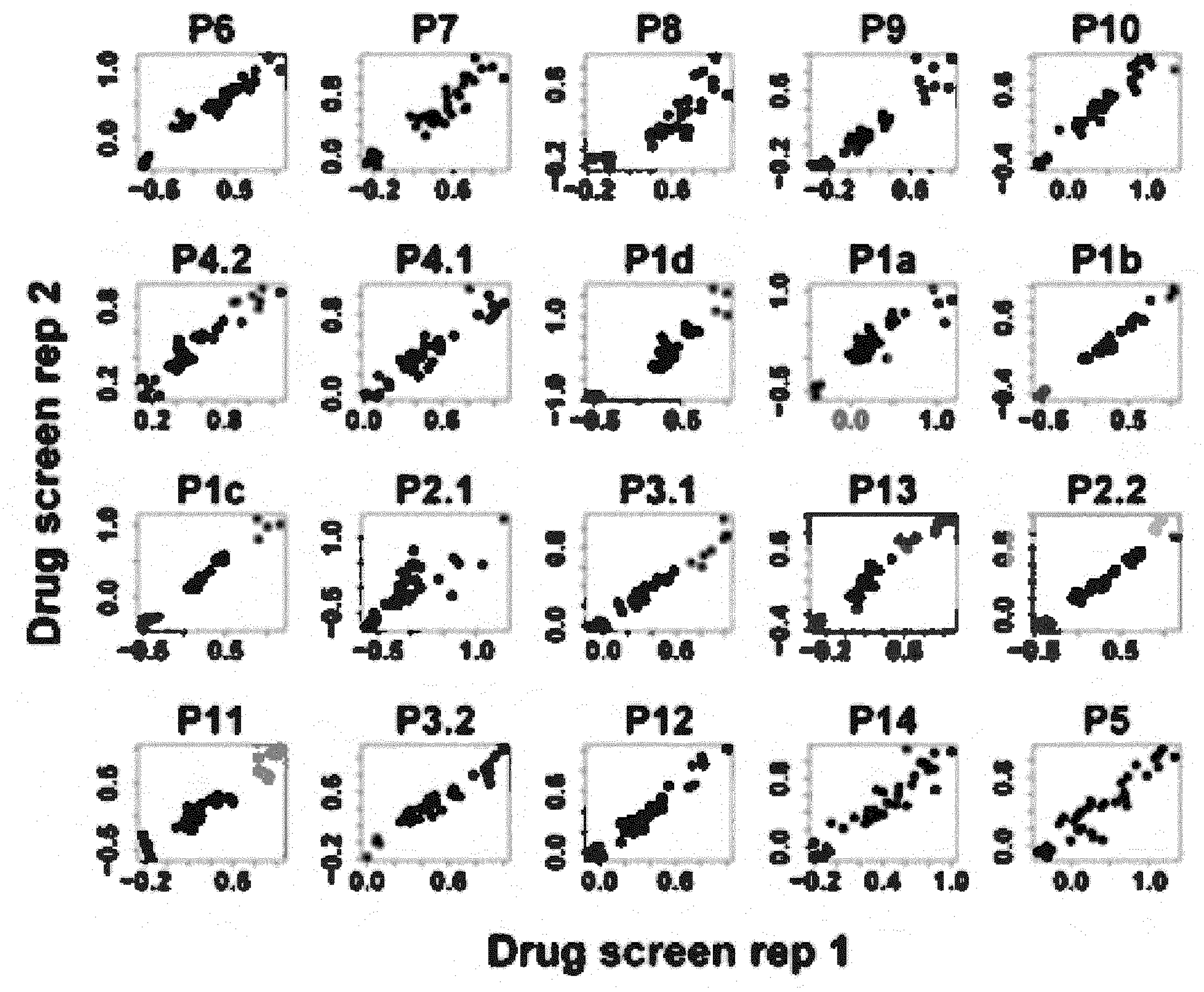
P6
P7
P8
P9
P10
P4.2
P4.1
P1d
P1a
P1b
P1c
P2.1
P3.1
P13
P2.2
P11
P3.2
P12
P14
P5
Drug screen rep 2
Drug screen rep 1

Fig. 3D

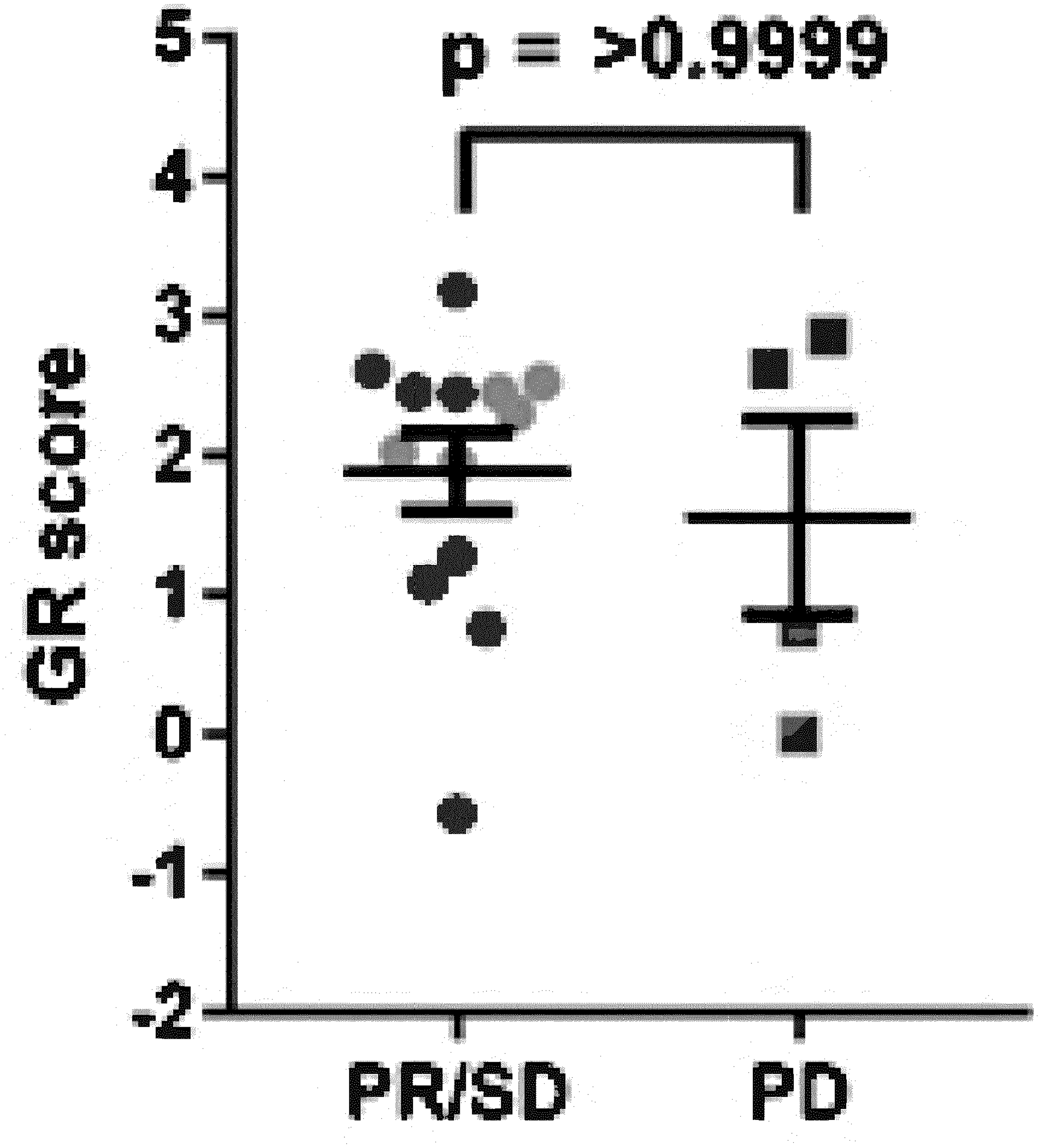

Fig. 3E

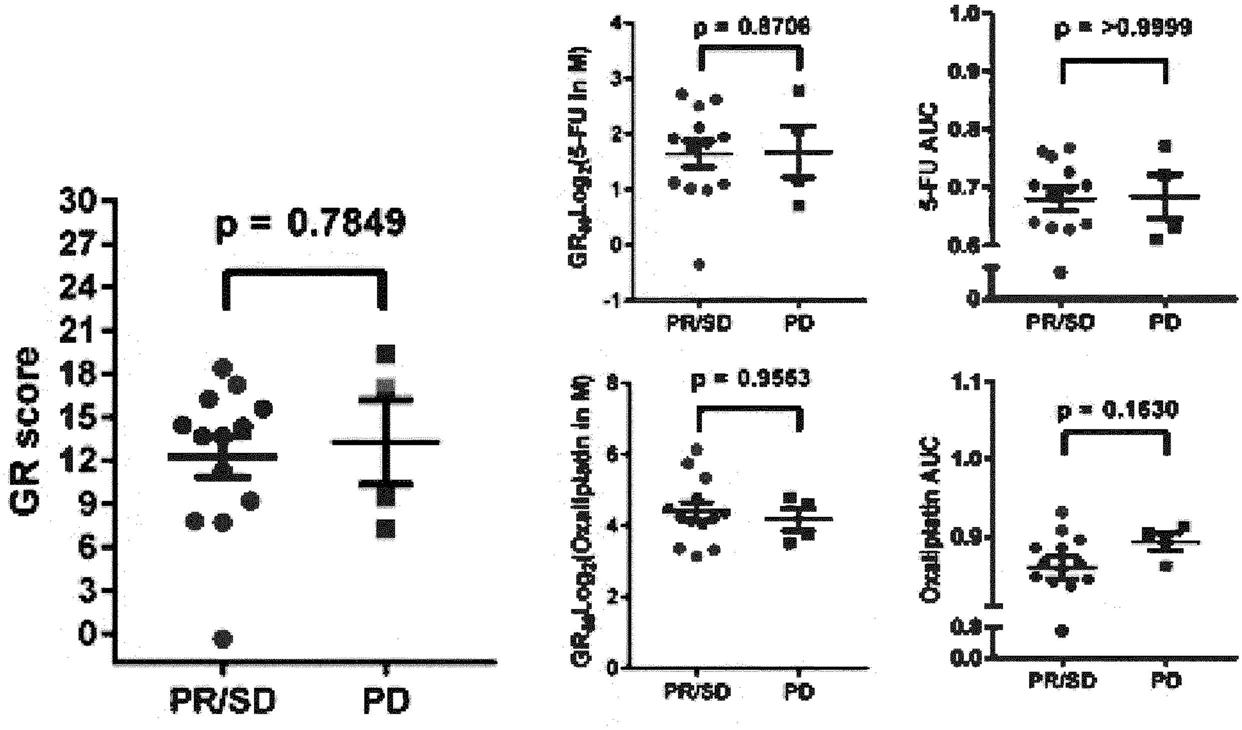
p = 0.7849
GR score
30
27
24
21
18
15
12
9
6
3
0
PR/SD
PD
p = 0.8706
$GR_{50}$ $Log_2$(5-FU in M)
PR/SD
PD
p = >0.9999
5-FU AUC
PR/SD
PD
p = 0.9563
$GR_{50}$ $Log_2$(Oxaliplatin in M)
PR/SD
PD
p = 0.1630
Oxaliplatin AUC
PR/SD
PD

Fig. 3F

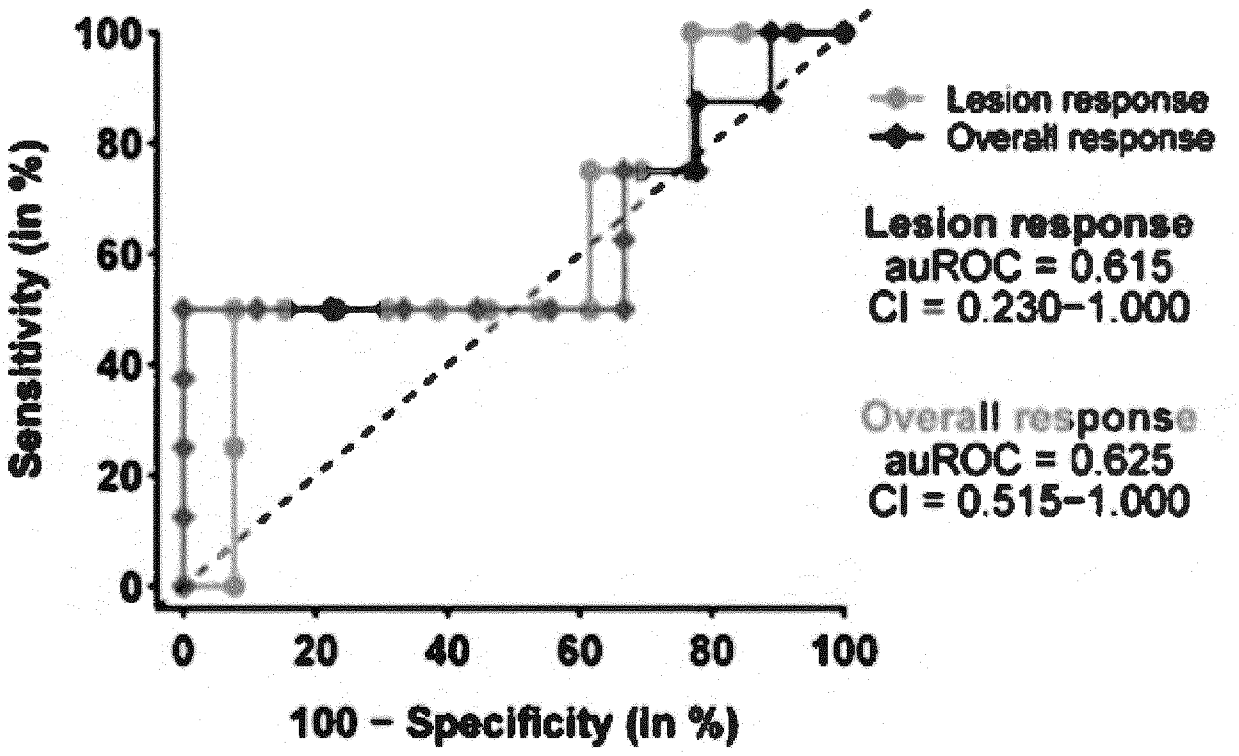
100
80
60
40
20
0
Sensitivity (in %)
0
20
40
60
80
100
100 − Specificity (in %)
Lesion response
Overall response
Lesion response
auROC = 0.615
CI = 0.230−1.000
Overall response
auROC = 0.625
CI = 0.515−1.000

Fig. 3G

| Variable | Response | auROC | 95% CI |
|---|---|---|---|
| Sub-matrix | Lesion | 0.6154 | 0.2686 to 0.9622 |
| Sub-matrix | Overall | 0.6250 | 0.3295 to 0.9205 |
| Whole matrix | Lesion | 0.5577 | 0.1653 to 0.9501 |
| Whole matrix | Overall | 0.5833 | 0.2876 to 0.8791 |
| $GR_{50}$ 5-FU | Lesion | 0.6923 | 0.4388 to 0.9458 |
| $GR_{50}$ 5-FU | Overall | 0.6667 | 0.3937 to 0.9396 |
| $AUC_9$ 5-FU | Lesion | 0.5 | 0.1147 to 0.8853 |
| $AUC_9$ 5-FU | Overall | 0.5972 | 0.3157 to 0.8787 |
| $GR_{50}$ Oxaliplatin | Lesion | 0.6923 | 0.4388 to 0.9458 |
| $GR_{50}$ Oxaliplatin | Overall | 0.6667 | 0.3937 to 0.9396 |
| $AUC_9$ Oxaliplatin | Lesion | 0.75 | 0.5003 to 0.9997 |
| $AUC_9$ Oxaliplatin | Overall | 0.6389 | 0.3601 to 0.9177 |

Fig. 4

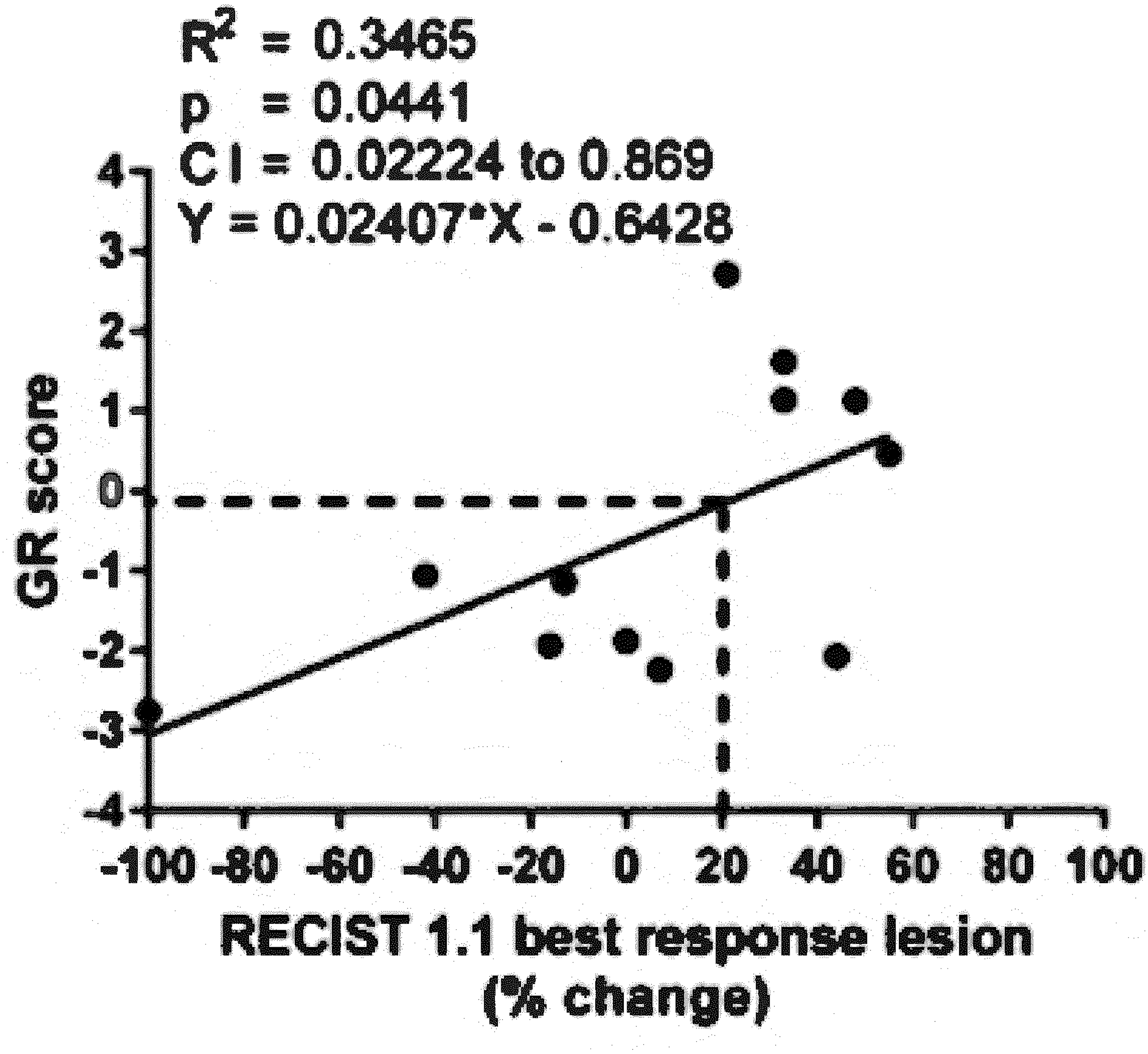
$R^2$ = 0.3465
p = 0.0441
C I = 0.02224 to 0.869
Y = 0.02407*X - 0.6428
GR score
4
3
2
1
0
-1
-2
-3
-4
-100 -80 -60 -40 -20 0 20 40 60 80 100
RECIST 1.1 best response lesion
(% change)

Europäisches Patentamt
European Patent Office
Office européen des brevets

# EUROPEAN SEARCH REPORT

Application Number
EP 18 20 7636

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GAO MEI ET AL: "Development of Patient-Derived Gastric Cancer Organoids from Endoscopic Biopsies and Surgical Tissues", ANNALS OF SURGICAL ONCOLOGY, RAVEN PRESS, NEW YORK, NY, US, vol. 25, no. 9, 12 July 2018 (2018-07-12), pages 2767-2775, XP036560660, ISSN: 1068-9265, DOI: 10.1245/S10434-018-6662-8 [retrieved on 2018-07-12] * the whole document * * abstract * * figure 5 * ----- | 1-14 | INV. G01N33/50 G01N33/574 |
| Y | GEORGIOS VLACHOGIANNIS ET AL: "Patient-derived organoids model treatment response of metastatic gastrointestinal cancers", SCIENCE, vol. 359, no. 6378, 23 February 2018 (2018-02-23), pages 920-926, XP55596283, US ISSN: 0036-8075, DOI: 10.1126/science.aao2774 * the whole document * * abstract * ----- | 1-14 | TECHNICAL FIELDS SEARCHED (IPC) G01N |
| Y | HIROSUMI TAMURA ET AL: "Evaluation of anticancer agents using patient-derived tumor organoids characteristically similar to source tissues", ONCOLOGY REPORTS, 18 June 2018 (2018-06-18), XP55596290, ISSN: 1021-335X, DOI: 10.3892/or.2018.6501 * the whole document * * abstract * ----- -/-- | 1-14 | |

1 The present search report has been drawn up for all claims

| Place of search | Date of completion of the search | Examiner |
|---|---|---|
| The Hague | 14 June 2019 | Jenkins, Gareth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 18 20 7636

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | TATSUYA USUI ET AL: "Establishment of a Novel Model for Anticancer Drug Resistance in Three-Dimensional Primary Culture of Tumor Microenvironment", STEM CELLS INTERNATIONAL, vol. 2016, 1 January 2016 (2016-01-01), pages 1-10, XP55596292, US ISSN: 1687-966X, DOI: 10.1155/2016/7053872 * the whole document * * abstract * ----- | 1-14 | |
| Y | MOHAMED ELBADAWY ET AL: "Development of an Experimental Model for Analyzing Drug Resistance in Colorectal Cancer", CANCERS, vol. 10, no. 6, 1 June 2018 (2018-06-01), page 164, XP55596303, CH ISSN: 2072-6694, DOI: 10.3390/cancers10060164 * the whole document * * abstract * * section 2 * ----- | 1-14 | TECHNICAL FIELDS SEARCHED (IPC) |

The present search report has been drawn up for all claims

1

| Place of search | Date of completion of the search | Examiner |
|---|---|---|
| The Hague | 14 June 2019 | Jenkins, Gareth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- NL 4900203114


### Non-patent literature cited in the description


- **LAZEBNIK, Y.** *Nature Reviews Cancer,* 2010, vol. 10, 232-233
- **BEKELE ; BRINDLEY.** *Clinical Lipidology,* 2012, vol. 7, 313-328
- **BOSCH et al.** *Clin. Cancer Res.,* 2016, vol. 22, 4612-22
- **YANG LIU et al.** *Cancer cell,* 2018, vol. 33, 721-35
- **WETERING et al.** *Cell,* 2015, vol. 161 (4), 933-945
- **WEEBER et al.** *PNAS,* 2015, vol. 112 (43), 13308-13311
- **VLACHOGIANNIS, G. et al.** *Science,* 2018, vol. 359, 920-926
- **TIRIAC, H. et al.** *Cancer Discov.,* 2018, vol. 8 (9), 1-18
- **EISENHAUER, E. A. et al.** *Eur. J. Cancer,* 2009, vol. 45, 228-247
- **HAFNER, M. et al.** *Nat. Methods,* 2016, vol. 13, 521-527
- **HARRIS, L. A. et al.** *Nat. Methods,* 2016, vol. 13, 497-500
- **GRABINGER, T. et al.** *Cell Death and Disease,* 2014, vol. 5, e1228
- **PAULI et al.** *Cancer Discov,* 2017, vol. 7 (5), 462-77
- **WEEBER, F. et al.** *PNAS,* 2015, vol. 112, 13308-11
- **LIANG-CHU, M. M. Y.** *PLoS One,* 2015, vol. 10, e0116218
- **LORIO, F. et al.** *Cell,* 2016, vol. 166, 740-754
- **GUGLIELMI, A. P. ; SOBRERO, A. F.** *Cancer Res.,* 2007, vol. 1, 57-63
- **TIRIAC, H. et al.** *Cancer Discov.,* 2018
- **BOSCH, L. J. W. et al.** *Clin. Cancer Res.,* 2016, vol. 22, 4612-22
- **ASCHELE, C. et al.** *J. Clin. Oncol.,* 1999, vol. 17, 1760-1760
- **EBERT, M. P. A. et al.** *N. Engl. J. Med.,* 2012, vol. 366, 44-53
- **HAAN, J. C. et al.** *Nat. Commun.,* 2014, vol. 5, 5457